# EUROPEAN PATENT SPECIFICATION

(11) **EP 3 383 444 B1**
(45) Date of publication and mention of the grant of the patent: **20.05.2020**
(21) Application number: 16820050.9
(22) Date of filing: 02.12.2016
(51) Int. Cl.: A61L 27/24, A61L 27/36, A61L 31/04

(54) **FILAMENTOUS GRAFT IMPLANTS AND METHODS OF THEIR MANUFACTURE AND USE**
FILAMENTÖSE TRANSPLANTATE UND VERFAHREN ZU DEREN HERSTELLUNG UND VERWENDUNG
IMPLANTS DE GREFFON FILAMENTEUX ET LEURS PROCÉDÉS DE FABRICATION ET D'UTILISATION

(30) Priority: 02.12.2015 US 201562262246 P
(43) Date of publication of application: 10.10.2018
(73) Proprietor: Cook Biotech Incorporated, West Lafayette, IN 47906 (US)
(72) Inventor: BRONIKOWSKI, Christine, M., Charleston, WV 25314 (US); OBERMILLER, F., Joseph, West Lafayette, IN 47906 (US); COLBURN, Wade, L., Lone Tree, Colorado 80124 (US)
(74) Representative: Stafford, Jonathan Alan Lewis
(86) International application number: PCT/US2016/064656
(87) International publication number: WO 2017/096188

(56) References cited:
- US-A- 6 165 198
- US-A1- 2001 016 205
- US-A1- 2009 287 308
- US-B2- 6 656 200

## Description

### BACKGROUND

Aspects of the present disclosure relate generally to medical implants. In some more particular embodiments, aspects of the present disclosure relate to long, filamentous grafts that can be implanted in patients at sites where tissue ingrowth is desired.

In medicine, there are often situations in which it is desired to fill a site with an implanted material. As one specific example, in some circumstances it is desired to fill a site within the vascular system of a patient with an implant material to cause occlusion at the site and/or to obliterate a defect such as an aneurysm. Historically in clinical practice, occlusive implants have taken the form of metallic coils which occupy space at the site and promote embolization. Such metallic coils have been in use for many years and while other implant designs have been suggested, none have achieved the acceptance of coils. US6656200 B2 discloses an embolization device tor occluding a vessel. This device includes a matrix made of collagen and a radiopaque material, e.g. gold, iodinated organic compound. The matrix adopts a linear extended form (non-coiled)or a folded relaxed form such as a spherical, a random, or a cylindrically coiled form. The device can be introduced to a site for endovascular therapy through a catheter in its linear extended form. The diameter of the matrix can be 0.101 to 2.54 mm. The collagen can be of human or animal tissues. Segments of collagen fibers will be intact after dispersion and extrusion.

Cerebral aneurysms, also known as brain aneurysms, present particular challenges. These aneurysms can be accessed only using relatively small profile catheters, and their treatment with occlusive implants bears significant risks related to rupture or suboptimal defect filling and tissue development post-implant.

In view of this background, needs exist for improved and/or alternative implant materials as well as methods for their delivery. In certain of its aspects, the present disclosure is addressed to these needs.

### SUMMARY

In one aspect, the present disclosure relates to a filamentous tissue graft that includes an elongate filament body composed of one or more intact segments of a decellularized collagenous tissue membrane isolated from an animal source tissue. The filament body has a length of at least about 20 cm and a maximum cross-sectional dimension no greater than about 2 mm. In preferred embodiments, the filament body has a first filament body face and a second filament body face opposite to the first filament body face. The filament body also has a first filament body edge extending between the first filament body face and the second filament body face, and a second filament body edge opposite to the first filament body edge and extending between the first filament body face and the second filament body face. Further, the elongate filament body includes a laminate structure including a plurality of decellularized, intact collagenous tissue membrane layer segments laminated to one another, with each of the membrane layer segments including a first segment face a second segment face opposite the first segment face, a first segment edge, and a second segment edge opposite the first segment edge, wherein the first filament body face is provided by a first segment face of a first of said plurality of membrane layer segments, the first filament body edge is provided by the first segment edges of said plurality of membrane layer segments, and the second filament body edge is provided by the second segment edges of said plurality of membrane layer segments.

In another aspect, this disclosure provides a method for preparing a filamentous graft implant. The method includes providing a laminate structure including a plurality of decellularized, intact collagenous tissue membrane layer segments laminated to one another, and cutting from the laminate structure a filament body having a length of at least about 20 cm and a maximum cross-sectional dimension no greater than about 2 mm. In preferred forms, the cutting is conducted including laser cutting the laminate structure. Such laser cutting can provide a filament body having a first edge and an opposite second edge. The first and second edges can be constituted by edges of the tissue membrane layer segments, and can include a band of denatured collagen. The bands of denatured collagen at the first and second edges can each have a width that is about 5% to about 30% of the total width of the filament body. Additionally or alternatively, in certain embodiments, the cutting can include cutting a perforated line in the laminate structure which can later be torn to separate the filament body from the laminate structure. In these embodiments in which a perforated line is cut into the laminate structure, the resultant edges of the filament body can include protuberances, which are formed at the regions of the perforated line that are torn rather than cut. In certain modes, the edges with protuberances can have a saw-toothed configuration.

In still further embodiments, provided are apparatuses for delivering filamentous grafts into a patient that include a filamentous graft as described herein and a catheter having a lumen through which the graft is slidably deliverable. The apparatuses can also include a filamentous graft feeding device (e.g. a spool or bobbin) on which at least a portion of the filamentous graft is received, e.g. in a wound condition. Trailing portions of the filamentous graft are feedable from the feeding device, for example by unwinding, as leading portions of the filamentous graft are delivered distally through the lumen of the catheter.

Additional embodiments as well as features and advantages thereof will be apparent from the descriptions herein.

### BRIEF DESCRIPTION OF THE DRAWINGS

Figure 1 provides a perspective view of a filamentous graft partly received in a spooled configuration upon a spool.
Figure 2 provides a side view of the filamentous graft of Figure 1 in a straightened condition.
Figure 3 provides a cross-sectional view taken along line 2-2 of Figure 1 and viewed the direction of the arrows.
Figure 4 provides a top view of the filamentous graft of Figure 1.
Figure 5 provides a top view of an alternative filamentous graft having undulating edges.
Figure 6 provides a plan view of one cutting pattern for forming a filamentous graft from a sheet-form laminate structure.
Figure 7 provides a plan view of an alternate cutting pattern for forming a filamentous graft from a sheet-form laminate structure.
Figure 8 provides a plan view of an alternate cutting pattern for forming a filamentous graft from a sheet-form laminate structure.
Figure 9 provides a perspective view of a laser cutting operation for forming a filamentous graft from a tube-form laminate structure.
Figure 10 provides a schematic illustration of one embodiment of an apparatus and method for filling an aneurysm with a filamentous graft.
Figure 11 provides a chart illustrating blood clot times exhibited in the presence of certain embodiments of filamentous grafts, as described in Example 2.
Figures 12-31 provide digital images of various laser cut filamentous grafts, as described in Examples 3 and 4.

### DETAILED DESCRIPTION

While the present invention may be embodied in many different forms, for the purpose of promoting an understanding of the principles of the present invention, reference will now be made to embodiments, some of which are illustrated in the drawings, and specific language will be used to describe the same. It will nevertheless be understood that no limitation of the scope of the invention is thereby intended. Any alterations and further modifications in the described embodiments and any further applications of the principles of the present invention as described herein are contemplated as would normally occur to one skilled in the art to which the invention relates.

As disclosed above, embodiments of this disclosure relate to filamentous graft products and methods of their preparation and use. Additional embodiments of this disclosure relate to delivery apparatuses for filamentous grafts.

A filamentous graft as disclosed herein can have any suitable length. In some embodiments the filamentous graft will have a length of at least about 10 centimeters (cm), or of at least about 20 cm, or of at least about 50cm, or of at least about 1 meter. In certain embodiments, the filamentous graft will have a length in the range of about 20 cm to about 50 m, more typically in the range of about 20 cm to 30 m or about 20cm to about 5 m. It will be understood however that other lengths will also be suitable and that shorter grafts, for example having a length in the range of about 20 cm to about 5 m, can be cut from longer grafts, for example having a length in the range of about 10 m to about 50 m. Filamentous grafts herein can be of sufficient length that the delivery of a single such graft effects the desired treatment, or in other embodiments multiple filamentous grafts can be delivered to effect the desired treatment.

A filamentous graft as disclosed herein can also have any suitable cross-sectional dimension. In some embodiments, the filamentous graft will have a maximum cross-sectional dimension (taken perpendicular to the longitudinal axis of the filamentous graft) of less than about 2 mm, or of less than about 1 mm, or of less than about 0.5 mm. In certain embodiments, the filamentous graft will have a maximum cross-sectional dimension in the range of about 0.05 mm to about 1 mm, or in the range of about 0.05 mm to about 0.5 mm, or in the range of about 0.05 mm to about 0.25 mm. It will be understood that each of these described cross sectional dimensions can characterize the filamentous graft in addition to or as an alternative to the above-disclosed lengths for the filamentous graft.

A filamentous graft as disclosed herein can also have any suitable cross-sectional shape. For example, the filamentous graft can have a polygonal cross-sectional shape such as a rectangular, triangular, hexagonal or other polygonal cross-sectional shape. The filamentous graft can also have a continuous curved cross-sectional shape such as a circular or ovoid cross-sectional shape. As well, the filamentous graft may in certain embodiments have a cross-sectional shape that varies along its length, or that is the same along its length, or that is the same along at least a substantial portion (for example 80%, or 90%) of its length. In preferred embodiments, the filamentous graft will have a rectangular cross-sectional shape along a portion of or all of its length, which can be a square cross-sectional shape or another rectangular shape. These and other features of the cross-sectional shape of the filamentous graft can be suitably chosen by skilled persons in view of the disclosures herein.

When the filamentous graft has a rectangular cross-sectional shape, the graft may for example have lengths and maximum cross-sectional dimensions as disclosed above. Such rectangular cross-sectional shaped filamentous grafts can have suitable widths and thicknesses. Illustratively, the width of such grafts can be less than about 2 mm, or less than about 1 mm, or less than about 0.5 mm. Typically, the width will be in the range of about 0.05 mm to about 1 mm, or in the range of about 0.05 mm to about 0.5 mm, or in the range of about 0.05 mm to about 0.25 mm. Illustratively also, the thickness of such grafts can be less than about 2 mm, or less than about 1 mm, or less than about 0.5 mm. Typically, the thickness will be in the range of about 0.05 mm to about 1 mm, or in the range of about 0.05 mm to about 0.5 mm, or in the range of about 0.05 mm to about 0.25 mm.

The filamentous graft can have a substantially uniform cross-section along its length. Illustratively, the graft can have a maximum cross-sectional dimension that varies by no more than about 30%, or no more than about 20%, along the length of the graft or along at least a substantial portion (for instance at least 50%, or at least 80%) of the length of the graft in certain embodiments. In filamentous grafts that have a rectangular cross-sectional shape, the width and/or the thickness can vary by no more than about 30%, or no more than about 20%, along the length of the graft or along at least a substantial portion (for instance at least 50%, or at least 80%) of the length of the graft in certain embodiments.

The filamentous graft can also have beneficial strength properties. In some embodiments, the filamentous graft will have a tensile strength in its longitudinal direction that varies by no more than 50%, or no more than 40%, or no more than 30% along the length of the filamentous graft or at least a substantial continuous portion (e.g. at least 50%, or at least 80%) of the length of the filamentous graft. Such uniformity in longitudinal tensile strength can be tested, for example, by measuring the tensile strength of successive 5 cm lengths of the filamentous graft. It will be understood that the collagenous materials that are used in filamentous grafts herein are stronger when wetted to saturation with water, and that the tensile strengths disclosed herein are for the filamentous grafts when so wetted. Suitable conditions under which tensile strengths are measured include those in ASTM D3822 ("Standard Test Method for Tensile Properties of Single Textile Fibers') as published by ASTM International and in effect as of January 1, 2015.

As disclosed above, the filamentous grafts herein will include one or more intact segments of decellularized collagenous tissue membrane. Suitable materials for incorporation in any of the embodiments herein can be provided by membranous collagenous extracellular matrix (ECM) materials. For example, suitable membranous ECM materials include as examples those comprising submucosa, renal capsule membrane, dermal collagen, dura mater, pericardium, fascia lata, serosa, subserous fascia, amnion, peritoneum or basement membrane layers, including liver basement membrane. Suitable submucosa materials for these purposes include, for instance, intestinal submucosa including small intestinal submucosa, stomach submucosa, urinary bladder submucosa, and uterine submucosa. These or other ECM materials can be characterized as membranous tissue layers harvested from a source tissue and decellularized. These membranous tissue layers can have a porous matrix comprised of a network of collagen fibers, wherein the network of collagen fibers preferably retains an inherent network structure from the source tissue. In particular aspects, collagenous matrices comprising submucosa (potentially along with other associated tissues) useful in the present invention can be obtained by harvesting such tissue sources and delaminating the submucosa-containing matrix from smooth muscle layers, mucosal layers, and/or other layers occurring in the tissue source, and decellularizing the matrix before or after such delaminating . For additional information as to some of the materials useful in the present invention, and their isolation and treatment, reference can be made, for example, to U.S. Patent Nos. 4,902,508, 5,554,389, 5,993,844, 6,206,931, 6,099,567, 8,541,372 and 9,044,455.

Submucosa-containing or other ECM tissue, when used in the invention, is preferably highly purified, for example, as described in U.S. Patent No. 6,206,931 to Cook et al. Thus, preferred ECM material will exhibit an endotoxin level of less than about 12 endotoxin units (EU) per gram, more preferably less than about 5 EU per gram, and most preferably less than about 1 EU per gram. As additional preferences, the submucosa or other ECM material may have a bioburden of less than about 1 colony forming units (CFU) per gram, more preferably less than about 0.5 CFU per gram. Fungus levels are desirably similarly low, for example less than about 1 CFU per gram, more preferably less than about 0.5 CFU per gram. Nucleic acid levels are preferably less than about 5 µg/mg, more preferably less than about 2 µg/mg, and virus levels are preferably less than about 50 plaque forming units (PFU) per gram, more preferably less than about 5 PFU per gram. These and additional properties of submucosa or other ECM tissue taught in U.S. Patent No. 6,206,931 may be characteristic of any ECM tissue used in the present invention.

Submucosa-containing or other membranous ECM tissue material may retain one or more growth factors native to the source tissue for the tissue material, such as but not limited to basic fibroblast growth factor (FGF-2), transforming growth factor beta (TGF-beta), epidermal growth factor (EGF), cartilage derived growth factor (CDGF), and/or platelet derived growth factor (PDGF). As well, submucosa or other ECM materials when used in the invention may retain other bioactive agents native to the source tissue, such as but not limited to proteins, glycoproteins, proteoglycans, and glycosaminoglycans. For example, ECM materials may include native heparin, native heparin sulfate, native hyaluronic acid, native fibronectin, native cytokines, and the like. Thus, generally speaking, a submucosa or other ECM material may retain one or more native bioactive components from the source tissue that induce, directly or indirectly, a cellular response such as a change in cell morphology, proliferation, growth, protein or gene expression.

Submucosa-containing or other ECM materials can be derived from any suitable organ or other tissue source, usually sources containing connective tissues. The ECM materials processed for use in the invention will typically be membranous tissue layers that include abundant collagen, most commonly being constituted at least about 80% by weight collagen on a dry weight basis. Such naturally-derived ECM materials will for the most part include collagen fibers that are non-randomly oriented, for instance occurring as generally uniaxial or multiaxial but regularly oriented fibers. When processed to retain native bioactive factors, the ECM material can retain these factors interspersed as solids between, upon and/or within the collagen fibers. Particularly desirable naturally-derived ECM materials for use in the invention will include significant amounts of such interspersed, non-collagenous solids that are readily ascertainable under light microscopic examination with appropriate staining. Such non-collagenous solids can constitute a significant percentage of the dry weight of the ECM material in certain inventive embodiments, for example at least about 1%, at least about 3%, and at least about 5% by weight in various embodiments of the invention.

A submucosa-containing or other ECM material used in the present invention may also exhibit an angiogenic character and thus be effective to induce angiogenesis in a host engrafted with the material. In this regard, angiogenesis is the process through which the body makes new blood vessels to generate increased blood supply to tissues. Thus, angiogenic materials, when contacted with host tissues, promote or encourage the formation of new blood vessels into the materials. Methods for measuring in vivo angiogenesis in response to biomaterial implantation have recently been developed. For example, one such method uses a subcutaneous implant model to determine the angiogenic character of a material. See, C. Heeschen et al., Nature Medicine 7 (2001), No. 7, 833-839. When combined with a fluorescence microangiography technique, this model can provide both quantitative and qualitative measures of angiogenesis into biomaterials. C. Johnson et al., Circulation Research 94 (2004), No. 2, 262-268.

Further, in addition or as an alternative to the inclusion of such native bioactive components, non-native bioactive components such as those synthetically produced by recombinant technology or other methods (e.g., genetic material such as DNA), may be incorporated into the ECM material, e.g. before and/or after the ECM material is cut to form a filamentous graft herein. These non-native bioactive components may be naturally-derived or recombinantly produced proteins that correspond to those natively occurring in an ECM tissue, but perhaps of a different species. These non-native bioactive components may also be drug substances. Illustrative drug substances that may be added to materials include, for example, anti-clotting agents, e.g. heparin, antibiotics, anti-inflammatory agents, thrombus-promoting substances such as blood clotting factors, e.g., thrombin, fibrinogen, and the like, and anti-proliferative agents, e.g. taxol derivatives such as paclitaxel. The non-native bioactive component(s) can also be cells, including for example stem cells, which can be attached to and/or cultured on the ECM of filamentous grafts prior to implantation if desired. These and/or other non-native bioactive components can be incorporated into and/or onto ECM material in any suitable manner, for example, by surface treatment (e.g., spraying) and/or impregnation (e.g., soaking), just to name a few. Also, these substances may be applied to the ECM material in a premanufacturing step (e.g. applied to the filamentous graft), immediately prior to the procedure (e.g., by soaking the filamentous graft material in a solution containing a suitable antibiotic such as cefazolin), or during or after engraftment of the filamentous graft material in the patient.

In certain embodiments, a construct from which a filamentous graft is created can include a laminate of two or more individual layers of membranous ECM material (e.g., 2 or more layers bonded together). The total thickness of such a construct can in some forms be more than about 400 microns, or more than about 600 microns, or more than about 800 microns, or more than about 1,000 microns, or more than about 1,200 microns, or more than about 1,500 microns but typically less than about 2,000 microns. In certain aspects, the thickness of such a construct is in the range of about 200 microns to about 4,000 microns. Also in certain aspects, 2 to about 20 layers of membranous ECM tissue material are bonded in a laminate construct for use in creating a filamentous graft by cutting or otherwise forming the filamentous graft from the construct, more preferably 4 to about 16 layers.

Suitable bonding techniques in forming laminate constructs include chemical crosslinking and techniques other than chemical crosslinking, or combinations thereof. Techniques other than chemical cross-linking include vacuum pressing, lyophilization, or other dehydrothermal bonding conditions and/or the use of an adhesive, glue or other bonding agent. Suitable bonding agents may include, for example, collagen gels or pastes, gelatin, fibrin glues, or other agents including reactive monomers or polymers, for example cyanoacrylate adhesives. Bonding by chemical crosslinking can achieved using chemical cross-linking agents, such as glutaraldehyde, formaldehyde, epoxides, genipin or derivatives thereof, carbodiimide compounds, polyepoxide compounds, or other similar agents. The combination of dehydration-induced bonding and chemical crosslinking is used in certain embodiments.

A variety of dehydration-induced bonding methods can be used to fuse and thereby laminate layers of membranous ECM materials together. In one preferred embodiment, multiple layers of the membranous ECM material are compressed under dehydrating conditions. The term "dehydrating conditions" can include any mechanical or environmental condition which promotes or induces the removal of water from the multi-layered medical material. To promote dehydration of the compressed material, at least one of the two surfaces compressing the matrix structure can be water permeable. Dehydration of the material can optionally be further enhanced by applying blotting material, heating the matrix structure or blowing air, or other inert gas, across the exterior of the compressing surfaces. Particularly useful methods of dehydration bonding the ECM layers to one another include lyophilization, e.g. freeze-drying or evaporative cooling conditions, vacuum pressing, oven drying and/or air drying.

It is advantageous in some aspects of the invention to perform drying operations under relatively mild temperature exposure conditions that minimize potential deleterious effects upon the ECM materials of the invention, for example native collagen structures and potentially bioactive substances present. Thus, drying operations conducted with no or substantially no duration of exposure to temperatures above human body temperature or slightly higher, say, no higher than about 38° C, will preferably be used in some forms of the present invention. These include, for example, vacuum pressing operations at less than about 38° C, air drying at less than about 38° C, or either of these processes with no active heating - at about room temperature (about 25° C) or with cooling. These relatively low temperature conditions also include lyophilization conditions.

ECM materials for use in the invention can be processed using methods that decrease the content of undesired components of the source tissue such as cells, nucleic acid, lipids and/or immunoglobulins such as IgA, while retaining substantial levels of desired components from the source tissue such as growth factor(s) (e.g. Fibroblast Growth Factor-2), proteoglycans and/or glycosaminoglycans (GAGs). Such treatments can be performed with detergent, basic medium, liquid organic solvent, and/or disinfecting solution, for example as described in U.S. Patent No. 8,192,763 issued June 5, 2012.

Further, expanded membranous ECM materials for use in preparing filamentous graft materials as described herein can be formed by the controlled contact of an ECM material with one or more alkaline substances until the material expands, and the isolation of the expanded material. Illustratively, the contacting can be sufficient to expand the ECM material to at least 120% of (i.e. 1.2 times) its original bulk volume, or in some forms to at least about two times its original volume. Upon such expansion processing, the naturally-occurring intramolecular cross links and naturally-occurring intermolecular cross links of the ECM can be retained in the processed material sufficiently to maintain the material as an intact membranous sheet material; however, collagen fibrils in the collagenous sheet material can be denatured, and the membranous sheet material can have an alkaline-processed thickness that is greater than the thickness of the starting material, for example at least 120% of the original thickness, or at least twice the original thickness. Suitable alkaline substances for expansion of the membranous ECM material can include, for example, salts or other compounds that that provide hydroxide ions in an aqueous medium. Preferably, the alkaline substance comprises sodium hydroxide (NaOH). Illustratively, the concentration of the alkaline substance for treatment of the remodelable material can be in the range of about 0.5 to about 2 M, or about 0.5 to 4 M, with a concentration of about 1 M to about 3 M commonly being used. Additionally, the pH of the alkaline substance can in certain embodiments range from about 8 to about 14. In preferred aspects, the alkaline substance will have a pH of from about 10 to about 14, and most preferably of from about 12 to about 14. In addition to concentration and pH, other factors such as temperature and exposure time will contribute to the extent of expansion. Given the teachings herein these factors can be controlled to provide suitable expanded ECM materials that can be used to form filamentous grafts as described herein.

In certain embodiments filamentous grafts herein will have characteristic face and edge features. For example, one or both faces of the graft (which occur across the width of the graft) can be provided by a face of a decellularized tissue membrane segment or segments used to form the graft. Where the thickness of such a graft is constituted from a single tissue membrane segment, the first and second faces of the graft can be provided by the first and second opposed faces of the tissue membrane segment. Where the thickness of such a graft is constituted from a laminate of two or more tissue membrane segments that are bonded to one another, a first face of the graft can be provided by a face of a first tissue membrane segment and a second face of the graft opposite the first face of the graft can be provided by a face of a second tissue membrane segment. It will be understood that in such laminate form filamentous grafts, the first membrane segment and the second membrane segment, which provide the opposed faces of the graft, can be directly bonded to one another (e.g. in the case of a two-layer laminate) or can be indirectly bonded to one another through one or more tissue membrane segments occurring between the first membrane segment and the second membrane segment in the laminate (e.g. in the case of a three or more layer laminate, such as a three-layer, four-layer, five-layer, or six-layer laminate). In these regards, it will be understood that the first and second tissue membrane segments and any other tissue membrane segments can be provided by separate discrete pieces of tissue membrane, or by a single piece of tissue membrane that is folded or otherwise gathered onto itself to provide tissue membrane segments in the described positions relative to one another.

As noted above, the filamentous grafts can also have characteristic edge features. Thus, in some embodiments in which one or more tissue membrane segments is used to form the graft, one or more edges of the graft can be provided by one or more edges of the tissue membrane segment(s). For example, in embodiments in which the graft has a thickness constituted from a single tissue membrane segment, first and second opposed edges of the graft can be provided by first and second opposed edges of the tissue membrane segment. Where the thickness of such a graft is constituted from a laminate of two or more tissue membrane segments that are bonded to one another, a first edge of the graft can be provided by adjacently stacked first edges of the two or more tissue membrane segments and a second edge of the graft opposite the first edge of the graft can be provided by adjacently stacked second edges of the two or more tissue membrane segments.

In addition or alternatively, the edges of the filamentous graft can have other features. In certain embodiments, the edges of the filamentous graft will be generally smooth and continuous along the length of the graft or a long at least a substantial continuous portion (e.g. at least 50%, or at least 80%) of the length of the graft. In other embodiments, the edges of the filamentous graft will be irregular in shape. For example, the edges of the filamentous graft may undulate along the length of the graft as provided by an undulating width of the graft. In certain forms, the edges of the graft form protuberances or peaks along the length of the graft. Such protuberances or peaks can contribute to frictional forces beneficial in the implantation and/or entanglement of the filamentous grafts in use.

As discussed in more detail below, in certain modes of manufacture, collagenous material at the edges of the filamentous grafts will be altered. This can provide filamentous grafts which have collagenous material that differs at edges of the grafts as compared to collagenous material spaced from edges of the grafts. In certain embodiments, the filamentous graft will have peripheral regions extending to edges of the graft that contain increased levels of denatured collagen as compared to an intermediate region located between the peripheral regions. In some forms, each of the peripheral regions will constitute at least about 3%, or at least about 5%, or at least about 10%, of the width of a filamentous graft having a rectangular cross-section, and typically each of the peripheral regions will constitute in the range of about 3% to about 35% of the width of a filamentous graft having a rectangular cross-section. Correspondingly, the above-noted intermediate region can constitute no more than about 94%, or no more than about 90%, or no more than about 80% of the width of a filamentous graft having a rectangular cross-section, and typically the intermediate region will constitute in the range of about 94% to about 30% of the width of a filamentous graft having a rectangular cross-section. It will be understood, however, that other relative sizes for the peripheral and intermediate regions are also possible.

Filamentous grafts herein can have a graft body that has been chemically crosslinked or that has not been chemically crosslinked. Filiamentous graft bodies that have been chemically crosslinked can be provided by chemically crosslinking a sheet material (e.g. a chemically crosslinked laminate as discussed above) from which the graft bodies are subsequently cut, and/or by chemically crosslinking the graft bodies after they have been cut from a sheet material (e.g. a non-chemically crosslinked laminate sheet as discussed above). As examples, chemical crosslinking agents for these purposes can be aldehydes (e.g. glutaraldehyde or formaldehyde), epoxides, genipin or derivatives thereof, carbodiimide compounds, polyepoxide compounds, or other similar chemical crosslinking agents.

Filamentous grafts herein can also include an imageable contrast agent or material. The imaging contrast agent or material can permit visualization of the filamentous grafts, or portions thereof, using externally-applied imaging techniques such as X-ray or magnetic resonance imaging (MRI) techniques. In some embodiments, the filamentous grafts include a radiopaque material. The radiopaque material can enable visualization of the graft by fluoroscopy or other X-ray imaging techniques. In certain embodiments, the collagenous ECM material from which the grafts are made, or at least a portion thereof, can be impregnated with an X-ray contrast agent or an MRI contrast agent. When used, the X-ray contrast agent can in some forms be an iodinated organic compound. Numerous iodinated organic radiopaque imaging agents are known and can be used for these purposes. These include, for example, commercially available X-ray contrast agents such as Diatrizoate (Hypaque), Ioxaglate (Hexabrix), Metrizoate (Isopaque), Iopamidol (Isovue), Iohexol (Omnipaque), Ioxilan (Oxilan), Iopromide (Ultravist), and Iodixanol (Visipaque). When used, the MRI contrast agent can include a paramagnetic substance, such as gadolinium. Numerous MRI contrast agents are known and can be used, including for example Gadocoletic acid, Gadomelitol, Gadomer 17, gadoterate (Dotarem), gadodiamide (Omniscan), gadobenate (MultiHance), gadopentetate (Magnevist, Magnegita, Gado-MRT ratiopharm), gadoteridol (ProHance), gadoversetamide (OptiMARK), gadoxetate (Primovist), gadobutrol (Gadovist), gadofosveset (Ablavar, formerly Vasovist), and gadoxetate (Eovist). These or other contrast agents in liquid form, typically as solutions or suspensions of the contrast agent molecule, can be soaked into one or more of the ECM layers used in forming the structure or construct to be cut to form the filamentous graft. In certain embodiments, one or more layers soaked in the contrast agent are sandwiched in between layers that are not soaked in the substances. The formed construct can then be dried, processed and cut as described herein to form a filamentous graft that is visible by X-ray, MRI or other applied imaging techniques. Alternatively, a cut filamentous graft as described herein can be soaked with a solution or suspension of the contrast agent, and then dried. Still further, radiopaque pastes (e.g. containing iodinated polymers) can be spread between layers of the construct to be cut to form the filamentous graft. In other embodiments, radiopaque metallic materials such as powders, wires, or films, can be embedded between layers of the ECM construct cut to form the filamentous graft, to provide filamentous grafts with the radiopaque metallic materials embedded therein. The metallic materials can for example be gold, tantalum, barium or other radiopaque metallic substances. In still further embodiments, a filamentous ECM graft as described herein can be twisted with, woven with, or otherwise combined with an elongate radiopaque, paramagnetic or superparamagnetic article such as a metallic (e.g. gold) wire to form an overall elongate graft, in some embodiments a multifilament graft, that is visible under X-ray. These and other modes of rendering the filamentous ECM graft material imageable using an externally-applied imaging technique will be available to those of skill in the art given the descriptions herein.

In certain forms, filamentous grafts herein are coated and/or impregnated with a polymeric substance that prevents or retards leaching of a substance incorporated in the grafts, for example a contrast agent as discussed above, from the grafts when they are contacted with an aqueous medium such as water or blood. Coating and/or impregnating by dipping, spraying or other means will be suitable for these purposes. In preferred aspects, the polymeric substance comprises or is constituted from one or more biodegradable polymers. Suitable biodegradable polymers for these purposes include, for example, as polylactic acid, polyglycolic acid or copolymers thereof, polyanhydride, polycaprolactone, polyhydroxy-butyrate valerate, polyhydroxyalkanoate, or another biodegradable polymer or mixture thereof. These or other suitable polymers can be dissolved in a suitable solvent, and the resulting solution applied to the filamentous graft by dipping, spraying or other techniques, either along with the contrast agent (e.g. in the same solution or suspension) and/or after application of the contrast agent to the filamentous graft. The graft can then if desired be dried to form a dried graft having the polymer(s) coated on and/or impregnated in the graft. When the leachable substance is a contrast agent, for example any of those discussed above, the polymeric substance can prevent or retard leaching of the contrast agent and thereby increase the duration for which the filamentous graft can be visualized using an externally applied imaging technique such as an X-ray or MRI imaging technique.

Filamentous grafts as described herein can be used alone as single filament implants, or can be combined with one or more additional filaments by weaving, braiding, twisting or otherwise to prepare multifilament implants. Illustratively, two or more, or three or more, filamentous grafts as described herein can be combined with one another to form a multifilament graft, or one, two or more, or three or more, filamentous grafts as described herein can be combined with one, two or more, or three or more, other types of filaments (including e.g. one or more radiopaque metallic wire(s), for example gold wire(s)) to prepare a multifilament graft. The filaments of such a multifilament graft can be held together by being twisted and/or woven with one another, or by any other suitable means.

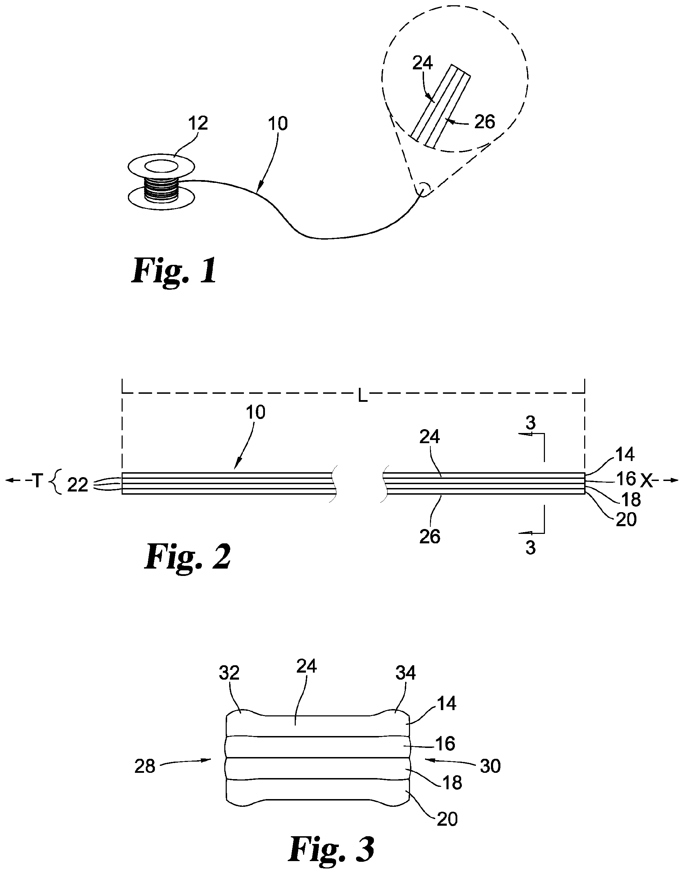

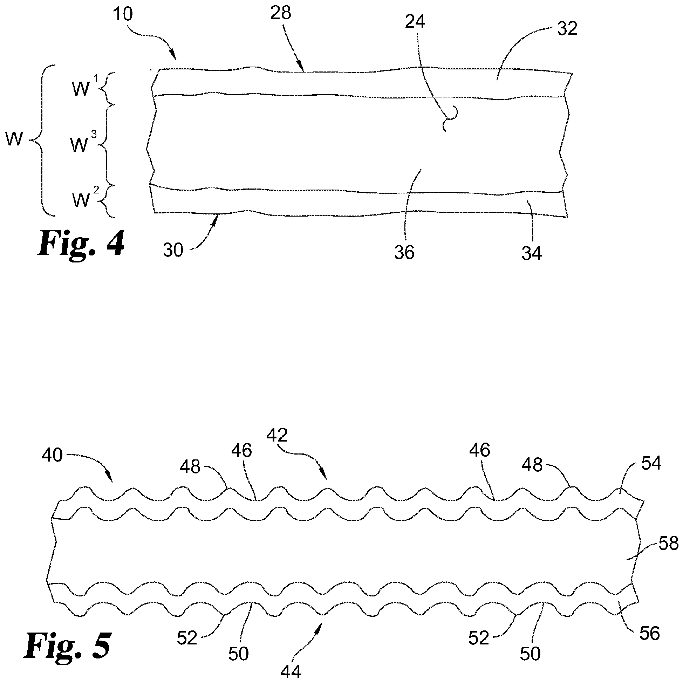

Referring now to Figures 1 to 4, shown is one illustrative embodiment of a filamentous graft. In Figure 1, filamentous graft 10 is shown partially wound on a spool 12. Figure 2 shows a side view of graft 10. Figure 3 shows a cross section of the graft 10 taken along line 3-3 of Figure 2 and viewed in the direction of the arrows. Figure 4 shows of top view of the graft 10 shown in Figure 2; and, in embodiments in which the graft 10 is the same on both top and bottom surfaces, can also represent a bottom view of the graft 10 shown in Figure 2.

Filamentous graft 10 is a laminate including a plurality of membranous ECM layers 14, 16, 18 and 20 laminated to one another, e.g. using any of the bonding materials or techniques disclosed herein. While four layers are shown, it will be understood that other numbers of layers can be used, including for example 2 to 20 layers, more preferably 4 to 16 layers. Bonding of the layers to one another forms bonded interfaces 22 between the adjacent layers. Filamentous graft 10 has a first side 24 formed by an outer exposed surface of layer 14 and a second side 26 opposite the first side 24 and formed by an outer exposed surface of layer 20. Filamentous graft 10 further has a first lateral edge 28 formed by respective first lateral edges of layers 14, 16, 18, and 20 and a second lateral edge 30 opposite the first lateral edge 28 and formed by respective second lateral edges of layers 14, 16, 18 and 20.

Filamentous graft 10 has a width W between first lateral edge 28 and second lateral edge 30, a thickness T between first side 24 and second side 26, and a length L along longitudinal axis X of the graft 10. In preferred forms, the average width W of the filamentary graft 10 is in the range of about 0.05 mm to about 0.5 mm, more preferably about 0.05 mm to about 0.25 mm; and/or the average thickness T of the filamentary graft is in the range of about 0.05 mm to about 1 mm, more preferably about 0.05 mm to about 0.5 mm; and/or the length "L" of the graft is at least about 20 cm, more preferably at least about 30 cm, and in certain embodiments in the range of about 20 cm to about 50 m, or about 20 cm to about 5 m. Further, in certain embodiments, the ratio of the average width W to the average thickness T is from about 1:4 to about 4:1, or about 1:2 to about 2:1, or about 1:1.5 to about 1.5:1.

When used in conjunction with a cannulated element, such as a catheter or needle, in a graft delivery system, the filamentary graft can have a maximum cross-sectional area that is less than the cross-sectional area of a lumen of the cannulated element through which the graft will be delivered. In certain embodiments of such delivery systems, the maximum cross-sectional area of the filamentary graft will be 90% or less that of the lumen, 80% or less that of the lumen, or 60% or less that of the lumen. In certain embodiments, the cross-sectional area will be in the range of about 30% to about 60% that of the lumen. In addition or alternatively, the filamentary graft can have a maximum cross-sectional dimension that is less than that of the lumen of the cannulated element. Typically, the filamentous grafts of the present invention will swell when saturated with an aqueous medium (as compared to the dry state), for example with the maximum cross-sectional dimension increasing by at least 20%, or at least 30%, and typically in the range of about 20% to 120%. It will be understood that these values for maximum cross-sectional area and maximum cross-sectional dimension apply to the filamentous graft when saturated with an aqueous medium, such as water or blood.

As discussed above, in certain forms, filamentous graft 10 will have a peripheral band of denatured collagenous material 32 extending inward from lateral edge 28 and a peripheral band of denatured collagenous material 34 extending inward from lateral edge 30, with an intermediate band of collagenous material 36 occurring between peripheral bands 32 and 34 and being different from the denatured material in peripheral bands 32 and 34 (e.g. with the collagenous material in intermediate band 36 being undenatured or less denatured than the collagenous material in peripheral bands 32 and 34). The average thickness T of the graft 10 in the regions of bands 32 and 34 can be greater than that of the graft 10 in the region of band 36, for example due to swelling or expansion of the collagenous material upon its denaturation, which may occur when the filamentous graft 10 is formed by cutting it from a larger construct using a laser or another cutting means that generates heat that denatures the collagenous material of the starting construct. The average width w¹ of band 32 and the average width w² of band 34 can be the same or different, and each can in certain variants of the graft be in the range of about 0.02 mm to about 0.15 mm, and/or can represent about 3% to about 35% of the width W of the graft 10. In addition or alternatively, the average width w³ of the intermediate band 36 can in certain variants of the graft 10 be in the range of about 0.04 mm to about 0.46 mm, and/or can represent about 30% to about 94% of the width W of the graft 10. Additional features of, and methods of making, graft 10 and similar grafts can include any of those features for filamentous grafts discussed herein.

Figure 5 shows a top view of another embodiment of a filamentous graft 40. Graft 40 can be the same as graft 10, except as discussed herein. Filamentous graft 40 has scalloped or otherwise undulating edges 42 and 44, as compared to the generally straight edges 28 and 30 of filamentous graft 10. Thus edge 42 of filamentous graft 40 is defined by a series of valleys 46 separated by peaks 48, and opposite edge 44 of filamentous graft 40 is also defined by a series of valleys 50 separated by peaks 52. In the illustrated embodiment, valleys 46 and 50 are generally rounded and concave, and peaks 52 are generally pointed and convex. It will be understood, however, that in additional embodiments undulating edges can be provided by other configurations, for example where valleys and/or peaks are generally pointed (e.g. "V" shaped) or rectangular. When subjected to delivery forces provided by flowing liquid or another fluid as discussed further below, the undulating edges of such filamentous grafts provide advantageous fluid contact surfaces benefiting the travel of the grafts with the flowing fluid.

As also shown in Figure 5, filamentous graft 40 can have an undulating peripheral band of denatured ECM material 54 extending inward from lateral edge 42 and an undulating peripheral band of denatured ECM material 56 extending inward from lateral edge 44, with an intermediate band of ECM material 58 occurring between peripheral bands 42 and 44 and being different from the denatured material in peripheral bands 42 and 44 (e.g. with the ECM material in intermediate band 58 being undenatured or less denatured than the ECM material in peripheral bands 54 and 56). The undulations of bands 54 and 56 can correspond to the undulations of edges 42 and 44, respectively. Additional properties of bands 54 and 56 can be the same as those for bands 32 and 34 of filamentous graft 10 discussed herein, and/or additional properties of band 58 can be the same as those for band 36 of filamentous graft 10 discussed herein.

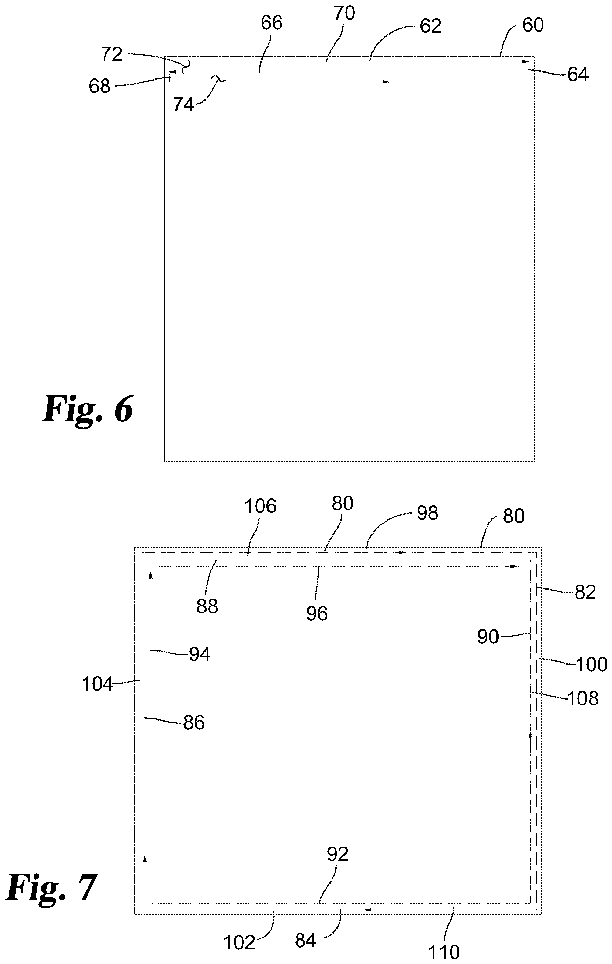

Filamentous grafts as described herein can be made in a variety of ways. Typically, the filamentous graft will be cut from a starting construct or structure. The starting structure can have any suitable size and shape to form the filamentous graft. In some forms, the starting structure is a sheet. When this is the case, any of a variety of cutting patterns may be employed. In certain modes of operation, a zig-zag or back-and-forth cutting pattern can be employed. In such modes, the direction of cutting can be periodically reversed, so that segments of the sheet that are adjacent to one another in a given, constant direction along the sheet sequentially form the length of the filamentous graft. Illustrative reference can here be made to Figure 6, which shows a sheet 60 from which a filamentous graft can be cut. Sheet 60 can be considered to have an X axis and a Y axis perpendicular to the X axis. In one mode of zig-zag cutting, a first cut 62 (dotted lines represent cutting paths) can be made traveling in the X axis in a first X direction for a distance across the sheet 60, after which a second cut 64, which is relatively short compared to the first cut 62, is made in the Y axis on the sheet 60. A third cut 66 is then made, in the X axis, and in a second X direction that is opposite to the first X direction. A fourth cut 68 is then made, in the Y axis in the same direction as the second cut 64. The zig-zag pattern provided by the first, second, third, and fourth cuts 62, 64, 66, and 68 is then repeated to form the filamentous graft. As will be understood, in this process, a filamentous graft in which sheet segments 70, 72 and 74 which are adjacent to one another in the Y axis of the sheet 60 (as well as other sheet segments in the Y axis similarly situated in the cutting pattern) occur sequentially is formed. In the specific illustrated embodiment, along the length of the filamentous graft, the adjacent Y axis segments 70, 72 and others within the zig-zag pattern, are separated by relatively shorter segments formed at the relatively shorter cuts (e.g. 64 and 68) in the Y axis. After the zig-zag cutting pattern is complete, or during execution of the zig-zag cutting pattern, the formed filamentous graft can be pulled to bring its shape to a relatively more straightened condition. For example, such pulling can include winding the formed filamentous graft under tension onto a mandrel or other device such as a spool or bobbin.

With reference now to Figure 7, illustrated is another method for creating a filamentous graft from a sheet-form starting structure 80. In particular, Figure 7 illustrates a spiraling cutting pattern for creating a filamentous graft from structure 80. A first cut 82 is made in the X axis of sheet 80. A second cut 84 extends from the end of first cut 82 in the Y axis of sheet 80. A third cut 86 extends from the end of second cut 84 in the X axis of sheet 80, and a fourth cut 88 extends from the end of the third cut 86 in the Y axis of sheet 80. Fourth cut 88 continues in the Y axis to a point short of and therefore not intersecting first cut 82, to leave a thickness of the material of structure 80 that approximately corresponds to the thickness desired for the filamentous graft. A fifth cut 90 extends from the end of fourth cut 88 in the X axis and extending generally parallel to first cut 82. Fifth cut 90 continues in the X axis to a point short of and therefore not intersecting second cut 84, to again leave a thickness of the material of structure 80 that approximately corresponds to the thickness desired for the filamentous graft. A sixth cut 92 extends from the end of fifth cut 90 in the Y axis of structure 80 and extending generally parallel to second cut 84. Sixth cut 92 extends to a point short of and therefore not intersecting third cut 86, to again leave a thickness of the material of structure 80 that approximately corresponds to the desired thickness for the filamentous graft. A seventh cut 94 extends from the end of sixth cut 92 in the X axis of structure 80 and extending generally parallel to third cut 86. Seventh cut 94 extends to a point short of and therefore not intersecting fourth cut 88, to again leave a thickness of the material of structure 80 that approximately corresponds to the desired thickness for the filamentous graft. An eighth cut 96 and subsequent cuts are made in an analogous spiraling fashion to create a filamentous graft from structure 80. As will be understood, in this process, a filamentous graft in which sheet segments 98, 100, 102, 104, 106, 108 and 110 (and others formed in the cutting pattern) which are alternately from the X axis and Y axis of the starting sheet structure 80 is formed. After the spiraling cutting pattern is complete, or during execution of the spiraling cutting pattern, the formed filamentous graft can be pulled to bring its shape to a relatively more straightened condition. For example, such pulling can include spooling the formed filamentous graft under tension onto a mandrel or other spooling device.

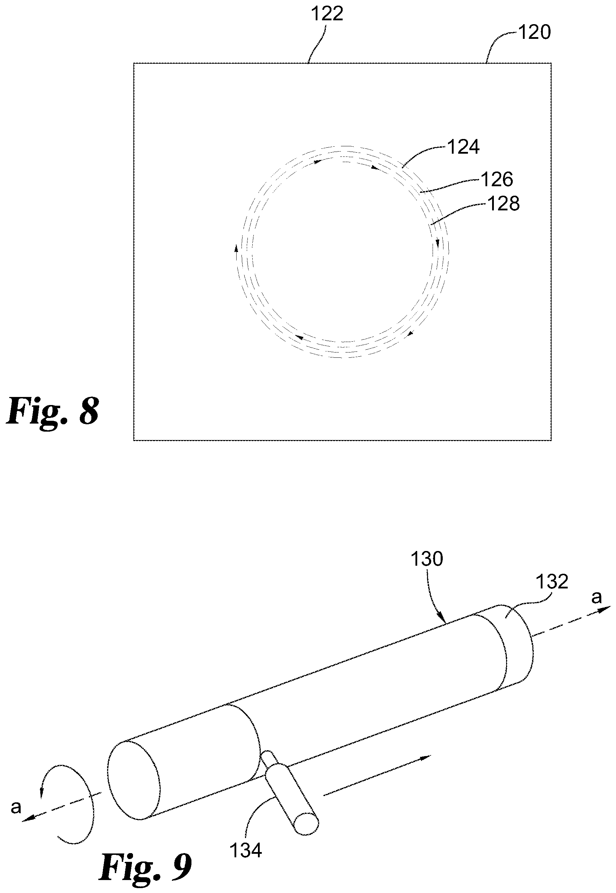

Figure 7 illustrates one embodiment of a spiraling cutting pattern that can be used to create a filamentous graft from a sheet structure. It will be understood that other spiraling cutting patterns can also be employed. For example, while Figure 7 illustrates a generally rectangular spiraling pattern in which subsequent cuts are oriented at 90° relative to previous cuts, other regular or irregular polygonal spiraling patterns can be used. Still further, continuously curving spiraling cutting patterns can be used to create filamentous grafts from starting sheet structures. For example, shown in Figure 8 is one such continuously curved spiraling cutting pattern, in which a continuous curved cut 122 is used to create a filamentous graft from starting structure 120. The formed filamentous graft therefore incorporates in sequence segments of the structure 120 from the outer regions of the cutting pattern to the inner regions of the cutting pattern. Illustratively, in Figure 8 the formed filamentous graft contains in sequence segments 124, 126 and 128 of structure 120. As with other embodiments herein, after the spiraling cutting pattern is complete, or during execution of the spiraling cutting pattern, the formed filamentous graft can be pulled to bring its shape to a relatively more straightened condition. For example, such pulling can include spooling the formed filamentous graft under tension onto a mandrel or other spooling device.

In certain preferred embodiments, a filamentous graft is cut from a tubular starting construct, which tubular construct can include one, two or more decellularized, intact tubular membranous collagenous structures isolated from tubular tissue sources, e.g. as in the case of small intestinal submucosal ECM). It has been found that cutting filamentous grafts from tubular starting structures is not only possible and convenient, but also can lead to filamentous grafts having advantageous characteristics. In these embodiments, a spiraling cut of the tube can be made about the axis of the tube to create the filamentous graft. Illustratively, with reference now to Figure 9, shown is a tubular starting structure 130 mounted around a cylindrical mandrel 132. To form a filamentous graft, mandrel 132 can be rotated on a lathe or other suitable device to thereby rotate starting structure 130 about its axis "a". During this rotation, a cutting device, for example a laser 134, can be moved linearly along the direction of longitudinal axis "a" to make a spiraling cut along structure that forms a filamentous graft. It will be understood that the rate of rotation of structure 130 and/or the rate of linear travel of laser 134 or other cutting implement (for example a blade) can be used to control the width of the resulting filamentous graft, if desired to provide a relatively constant width to the graft as disclosed for some preferred embodiments herein. The spiral-cut filamentous graft from the tube can retain a shape memory from the tubular shape of the starting structure, for example which leaves the filamentous graft wound around the mandrel at the completion of cutting. As with other embodiments herein, after the spiraling cutting pattern is complete, or during execution of the spiraling cutting pattern, the formed filamentous graft can be tensioned (e.g. pulled) to bring its shape to a relatively more straightened condition or a wound condition. For example, such pulling can include spooling the formed filamentous graft under tension onto a different mandrel or other spooling device.

It has been discovered that cutting filamentous grafts from tubular starting structures can be used to provide grafts having better uniformity in strength along their lengths than grafts cut using other methods for starting structures. For example, in grafts cut using patterns as disclosed in conjunction with Figures 6 and 7 discussed above, weaknesses in the formed filamentous graft can occur in locations corresponding to points at which the direction of cutting is sharply changed. In spiraling cut patterns as disclosed in conjunction with Figure 9 (and Figure 8), sharp directional changes in the cut are avoided, thereby giving filamentous grafts of greater uniformity in strength along their lengths.

Filamentous grafts as disclosed herein can be used in a variety of ways to treat human or non-human animal patients. Typically, the filamentous grafts will be delivered to an implant site in the patient using a delivery device. For example, typical delivery devices will include a lumen through which the filamentous grafts will be delivered, for example as in the case of a device including a catheter and/or a cannulated needle. When so delivered, the filamentous grafts during travel can be in a relatively straightened, longitudinally-extended condition during passage through the lumen. Upon exiting the lumen, the filamentous grafts can in some embodiments be forced to a gathered or compacted configuration. In such a configuration, the maximum cross-sectional dimension of the delivered graft can be smaller than the length of the filamentous graft in a straight condition, and typically much smaller, for example representing less than 10%, less than 5%, or less than 1%, of the length of the filamentous graft in a straight condition. These gathered or compacted, delivered configurations for the graft material can advantageously fill open spaces or add bulk to tissue regions.

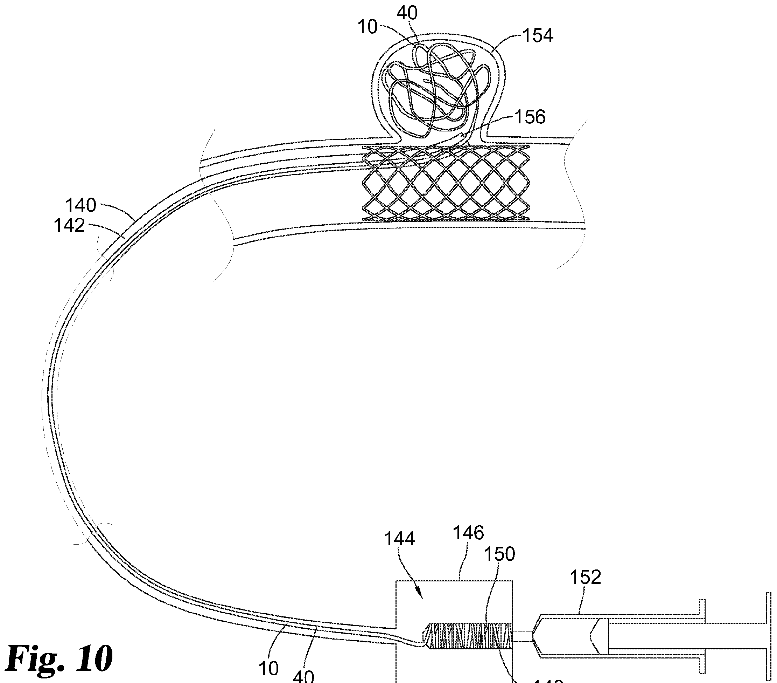

With reference now to Figure 10, shown is a schematic view of a filamentous graft delivery system in use. Such systems contemplated herein, including but not limited to that shown in Figure 10, can include a filamentous graft as disclosed herein, a feeding element on which the filamentous graft is retained (e.g. in wound condition), and a catheter or other cannulated device having a lumen through which the filament can be passed. As shown in Figure 10, filamentous graft 10,40 is delivered through a catheter 140 having a lumen 142 therethrough. Lumen 142 of catheter is fluidly coupled to an internal chamber 144 of a filamentous graft feeding device 146. Housed within internal chamber 144 is a filamentous graft feeding mechanism 148 having the filamentous graft 10,40 received thereon, for example in a wound condition. Feeding mechanism 148 can be any suitable mechanism for receiving thereon the filamentous graft 10,40 and for feeding the graft 10,40 through internal chamber 144 and into and distally through the lumen 142 of catheter 140 in response to a delivery force. In preferred forms the delivery force will comprise a pressurized stream of liquid, such as water, physiological saline or a liquid contrast agent such as an X-ray or MRI contrast agent that can soak into the filamentous graft to provide visualization of the graft after deployment in the patient, that passes distally through chamber 144 and into and through lumen 142, frictionally engaging filamentous graft 10,40, and driving a leading portion of the graft distally through lumen 142 which in turn causes trailing portions of the graft to feed off of feeding mechanism 148. In the illustrated embodiment, feeding mechanism 148 is a non-rotatable bobbin having its longitudinal axis aligned with the direction of flow of the pressurized liquid, with a wound portion 150 of the filamentous graft 10,40 wound therearound. The pressurized liquid is provided by a syringe 152 coupled to feeding device 146 and arranged to feed pressurized fluid distally through internal chamber 144 and into and through lumen 142 of catheter 140. It will be understood that other feeding mechanisms and arrangements are known and can also be used to feed or "pay off" the filamentous graft 10,40, including for example rotatable feeding mechanisms such as rotatable spools.

In certain uses, filamentous grafts as disclosed herein are delivered to sites in the vascular system of a human or other patient. In these uses, the grafts can serve to occlude a vessel and/or to obliterate a defect in a vessel. In one preferred use, illustrated in Figure 10, the filamentous grafts are delivered into an aneurysm 154 in a vascular vessel to partially or completely fill the aneurysm. In order to deliver the filamentous grafts to these or other vascular locations, the catheter 140 can be percutaneously introduced into the vascular system and a distal end 156 of the catheter 140 can be advanced to the site intended for delivery (e.g. within the aneurysm 154). The filamentous graft 10,40 can then be forced with the pressurized liquid through the lumen 142 of the catheter 140 and out of the distal end 156, and into the aneurysm or other site intended for delivery. The graft can, upon exiting the opening, form a gathered or compacted configuration as discussed above and as illustrated in Figure 10. Further, delivery of the graft 10,40 can in certain embodiments be used in conjunction with a device such as a stent 158 positioned across the opening or "neck" leading into the aneurysm 154, and the stent 158 or other device can serve to block or obstruct the opening or neck to facilitate holding or "jailing" the graft 10,40 within the aneurysm 154. For these purposes the stent 158 or other device can be delivered before or after the graft 10,40. When the stent 158 or other device is delivered before the graft 10,40, in certain forms, the distal end 156 of the catheter 140 can be sized such that can passes through a side opening of the implanted stent 158 or other device for delivery of the graft 10,40 into the aneurysm.

It will be understood that although certain preferred aspects described herein involve the delivery of the filamentous graft(s) through a catheter, other delivery instruments, such as cannulated needles, may also be used. Illustratively, the needles or other delivery devices can be used to deliver the filamentous graft(s) into a lumen or lumens of the vascular system, or into other openings or tissues in need of treatment, e.g. to repair, augment or fill an opening or tissue of a patient.

For the purpose of promoting a further understanding of aspects of the present disclosure, as well as features and advantages thereof, the following specific Examples are provided. It will be understood that these Examples are illustrative, and not limiting, of the disclosed embodiments herein.

### EXAMPLES

### Example 1

In this example, a number of different types of filamentous grafts were produced. Decellularized porcine small intestinal submucosa (SIS) was used as the graft material. This SIS material was processed in differing ways to make graft types as discussed below.

Six filamentous graft types were produced, numbered 1 to 6 below.

### 1. Lyophilized Filamentous Graft

Two tubes of wet, non-split (retaining their native tubular form), SIS were layered on an upright delrin mandrel (1" in diameter and 12" long, with the SIS material typically cut to about 9" in length.) A third non-split SIS tube is soaked in 1mL of Omnipaque 300 for 5 minutes and then layered onto the mandrel over the two previous SIS tubes. A fourth SIS tube is then layered over the third SIS tube. The resulting construct was processed in a vacuum press to dry for 8-10hrs. The dried construct is then put in a freezer for 10 minutes at -80 degrees C. The construct is removed from the freezer and immediately loaded into a laser cutter with an attachment to rotate the mandrel, and the SIS is laser cut into -0.010" wide filamentous grafts that are left on the mandrel. The entire mandrel is submerged in water for five minutes to fully rehydrate the SIS. The mandrel is then placed in the lyophilizer and the filamentous SIS graft is lyophilized.

### 2. Expanded Filamentous Graft

A bucket of wet SIS material (non-split tubes) was cut into approximately 18" (length) lengths. 3M NaOH solution was added to the bucket of material, and the bucket agitated for several minutes. The NaOH processing expands the SIS material. The expanded SIS material was then rinsed two times with water for fifteen minutes. 0.2M acetic acid was then added to the bucket and the bucket is agitated for fifteen minutes. The acetic acid solution was removed and the SIS material was rinsed several times in water for five minutes until a substantially neutral pH was reached.

Two of the expanded SIS tubes (12 inches in length) were then layered onto a 1" inch diameter delrin mandrel also 12 inches in length. A third non-split expanded SIS tube was soaked in 1mL of Omnipaque 300 for 5 minutes and then layered onto the mandrel over the two previous expanded SIS tubes. A fourth expanded SIS tube was then layered over the third SIS tube. This construct was then vacuum pressed, and the dried product laser cut in the same fashion as the Lyophilized Filamentous Graft #1 described above.

### 3. Double Lyophilized Filamentous Graft (FLX2)

Two layers of wet, non-split, SIS tubes were layered on an upright delrin mandrel (1" in diameter and 12" long, with the SIS tubes typically cut to about 9" in length.) A third SIS tube is soaked in 1mL of Omnipaque 300 for 5 minutes and then layered onto the mandrel. A fourth tube is added to the mandrel over the three previous tubes. The resulting construct is placed in a freezer for 10 minutes at -80 degrees C. The construct is removed from the freezer and immediately loaded into a laser cutter with an attachment to rotate the mandrel, and the SIS is laser cut into -0.010" wide filamentous grafts that are left on the mandrel. The entire mandrel was submerged in water for five minutes to fully rehydrate the SIS. The mandrel was then placed in the freezer at -80 degrees C for at least 4 hours, and the resulting product was lyophilized. The mandrel was then fully submerged in water for five minutes and then put through another cycle of freezing at -80 degrees C for at least four hours and then lyophilized.

### 4. EDC Crosslinked Expanded Filamentous Graft

A bucket of wet SIS material (non-split tubes) was cut into approximately 18" (length) lengths. 3M NaOH solution was added to the bucket of material, and the bucket agitated for several minutes. The NaOH processing expands the SIS material. The expanded SIS material was then rinsed two times with water for fifteen minutes. 0.2M acetic acid was then added to the bucket and the bucket is agitated for fifteen minutes. The acetic acid solution was removed and the SIS material was rinsed several times in water for five minutes until a substantially neutral pH was reached.

Two of the expanded SIS ("eSIS") tubes (12 inches in length) were then layered onto a 1" inch diameter delrin mandrel also 12 inches in length. A third non-split expanded SIS tube was soaked in 1mL of Omnipaque 300 for 5 minutes and then layered onto the mandrel over the two previous expanded SIS tubes. A fourth expanded SIS tube was then layered over the third SIS tube. This construct was then vacuum pressed, and the dried product laser cut in the same fashion as the Lyophilized Filamentous Graft #1 described above.

The laser cut filamentous graft was then crosslinked with EDC (1-ethyl-3-(3dimethylaminopropyl)carbodiimide hydrochloride) as follows. The delrin mandrel with the cut filamentous graft still thereon was soaked in EDC solution for 5 hours. The graft was then rinsed with water five times, by fully submerged the mandrel into a container of water. The devices are then submerged in a different container of water and soaked for two hours. The devices are soaked again in a different container of water for 16 hours. The filamentous grafts were then put in the -80 degrees C freezer for four hours and then lyophilized.

### 5. Crosslinked, Vacuum Pressed Filamentous Graft

Two tubes of wet, non-split (retaining their native tubular form), SIS were layered on an upright delrin mandrel (1" in diameter and 12" long, with the SIS material typically cut to about 9" in length.) A third non-split SIS tube is soaked in 1mL of Omnipaque 300 for 5 minutes and then layered onto the mandrel over the two previous SIS tubes. A fourth SIS tube is then layered over the third SIS tube. The resulting construct was processed in a vacuum press to dry for 8-10 hours. The dried product was laser cut in the same fashion as the Lyophilized Filamentous Graft #1 described above.

The delrin mandrel with the cut filamentous graft still thereon was thereafter soaked in EDC solution for 5 hours. The graft was then rinsed with water five times, by fully submerging the mandrel into a container of water. The devices are then submerged in a different container of water and soaked for two hours. The devices are soaked again in a different container of water for 16 hours. The filamentous grafts were then put in the -80 C freezer for four hours and then lyophilized.

### 6. Vacuum Pressed Filamentous Graft

Two layers of wet, non-split, SIS 2.0 were layered on an upright delrin mandrel (1" in diameter and 12" long, material typically cut to about 9" in length.) The third layer is soaked in 1mL of Omnipaque 300 for 5 minutes and then layered onto the mandrel. A fourth layer is added to the mandrel. The product is put in the vacuum press to dry for 8-10hrs. The dried product was laser cut in the same fashion as the Lyophilized Filamentous Graft #1 described above.

### Example 2

### Thrombogenicity Testing

Filamentous grafts #'s 3, 4, 5 and 6 prepared in Example 1 were subjected to testing to determine their thrombogenicity. In particular, samples were tested for clotting time using a method derived from the well-known Lee-White method. This was done according to weight and was 20mg per sample and controls were placed in 2mL siliconized tubes and heated to 37°C. 1mL of blood was added to each tube followed by capping the tube. The tube was inverted every 30 seconds until a clot formed, with continued heating at 37°C with a heating block in between inversions. The clot time was defined as the time point at which inversion of the tube did not lead to flow of the blood inside the tube. The clot time for each tube was recorded.

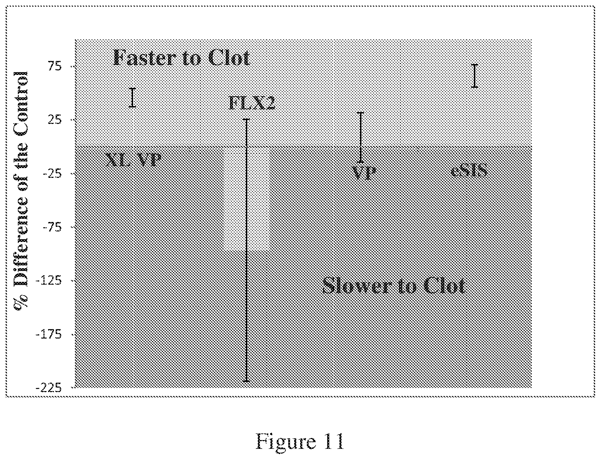

The results of this testing are shown in Figure 11, presented as a percent difference from the clot time of the control. As shown, the threads that are cross-linked with EDC, the XL VP (cross-linked VP thread, #5 in example 1) and eSIS (5eSIS with EDC, #4 in example 1), are pro-thrombogenic and faster to clot than normal blood. The FLX2 threads (#3 in Example 1) were rather neutral or slightly anti-thrombogenic. The Vacuum Pressed thread (#6 in Example 1) was found to have relatively the same thrombogenic properties as the control. For some clinical applications, such as for filling aneurysms, it may be beneficial to have a treatment that is neither highly pro-thrombogenic or highly anti-thrombogenic, which makes the Vacuum Pressed thread favorable when considered in this respect.

### Example 3

### Porosity Testing

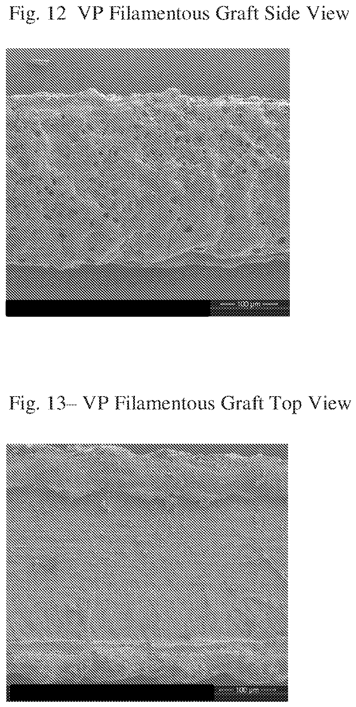

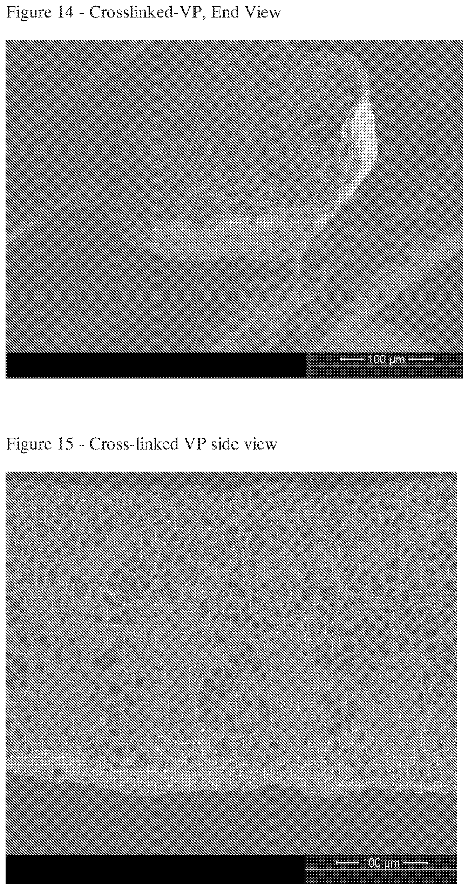

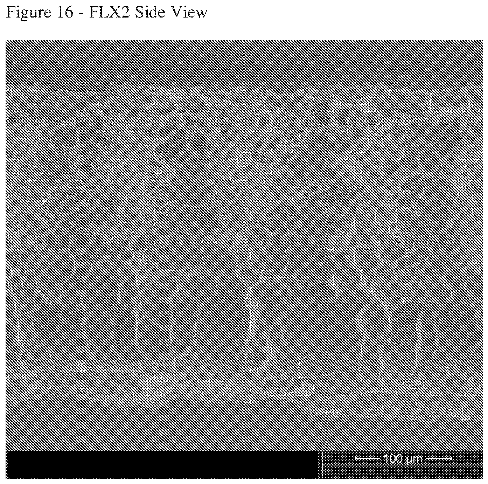

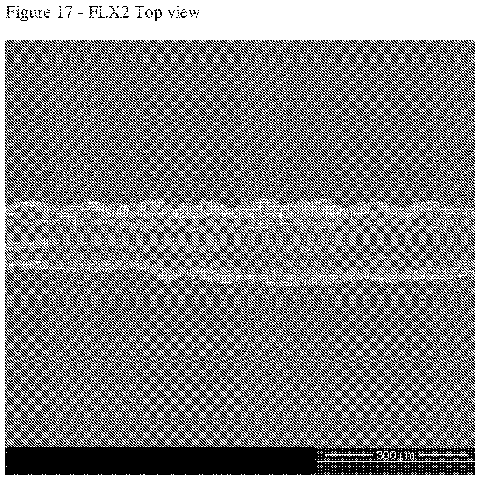

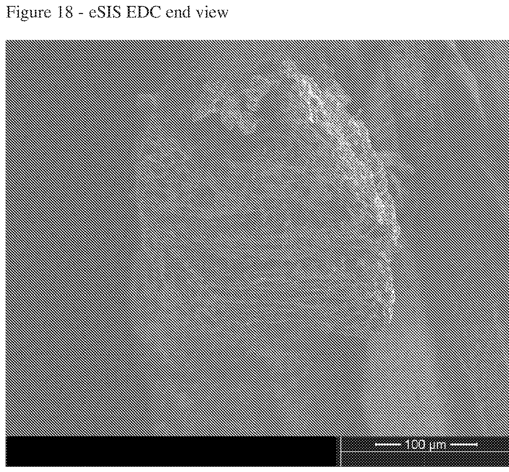

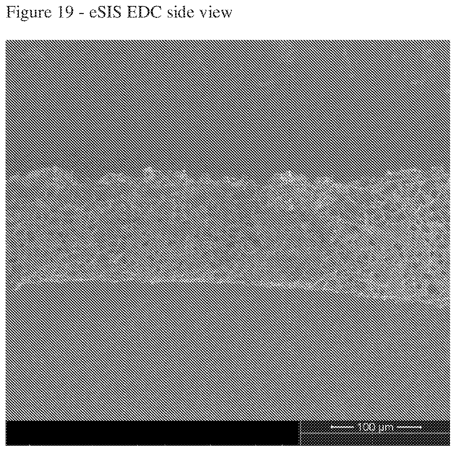

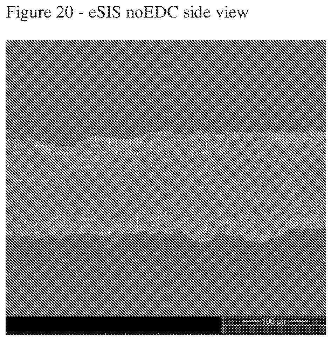

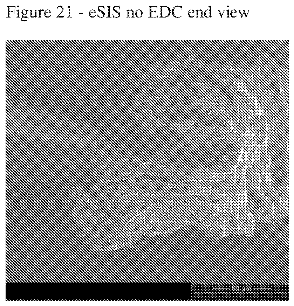

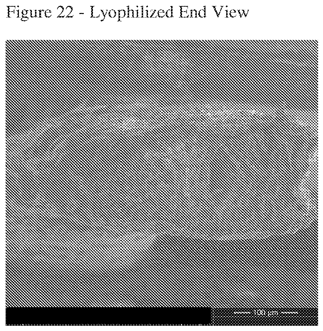

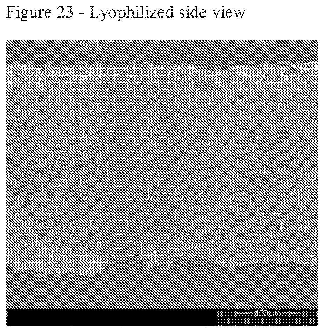

Samples of the filamentous graft materials prepared as in Example 1 were studied under a scanning electron microscope to assess porosity. Representative images for the graft types are provided in Figures 12 to 23. As shown, the heat denaturation effect from the laser at peripheral bands along the graft edges is seen the most in the vacuum pressed threads (see e.g. Figures 12 and 13). The native porous fiber structure of SIS is not apparent and is denatured or melted. This melting is also seen in the eSIS graft without EDC crosslinking (Figures 20-21). The cut grafts that are re-wetted and then lyophilized seem to undergo "reopening" or expansion of the pores (all other figures), but the top views still show the denatured peripheral bands still present. The end views of both crosslinked constructs (eSISEDC and VPXL) show an open, porous architecture.

### Example 4

### Investigation of Cutting Parameters

The purpose of this Example was to evaluate the effect that laser cutting has on the ECM material when it is cut into thin filaments, -.010" in width, and to determine how ECM processing and the laser settings affect the ECM. Two variables were investigated: the hydration state of the material during cutting (wet or dry) and the frequency of the laser pulse used to cut the material (high or low). All of the samples were made from 4-layer vacuum-pressed SIS material and were wrapped around a 1 and 1/8 inch diameter rod, with 4mL of Omnipaque 350 embedded in the material. In particular, to prepare the vacuum pressed sheet from which the filamentous grafts were cut, a sheet of SIS was laid flat and 4mL of Omnipaque 350 was coated on a middle region of the material. The Omnipaque was allowed to soak into the SIS for five minutes and then the material was wrapped around the rod. The samples were put in a vacuum press to dry for eight hours. The samples were then fully submerged in water for 5 minutes and then immediately cut with the laser with the listed settings. The dry samples were not hydrated prior to laser cutting.

There were four groups that were imaged: dry-low frequency (D-LF), wet-low frequency (W-LF), dry-high frequency (D-HF), and wet-high frequency (W-HF). The dry-low frequency settings were set to a laser speed of 83%, power of 10%, and a frequency of 325Hz. The dry-high frequency settings were the same power and speed, but set to a frequency of 550Hz. The wet-low frequency settings were set to a speed of 83%, power of 15%, and frequency of 325Hz. The wet-high frequency settings were the same power and speed, but set to a frequency of 550Hz. These samples were cut using an Epilog CO₂ laser and the commercially available Epilog rotary attachment.

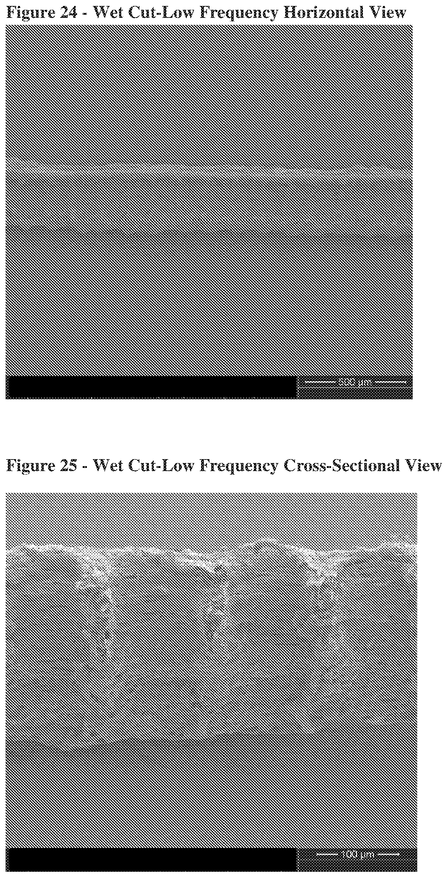

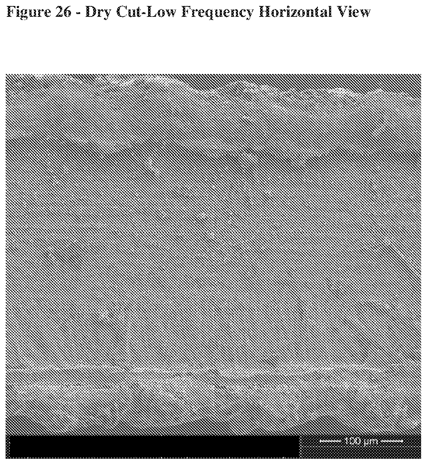

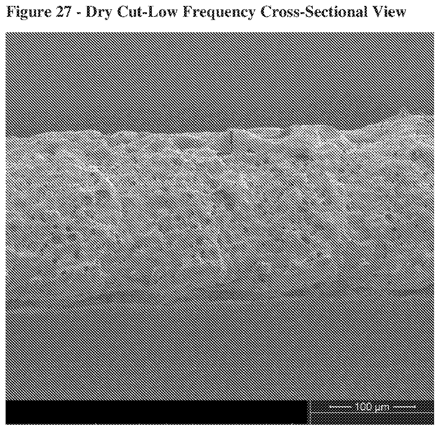

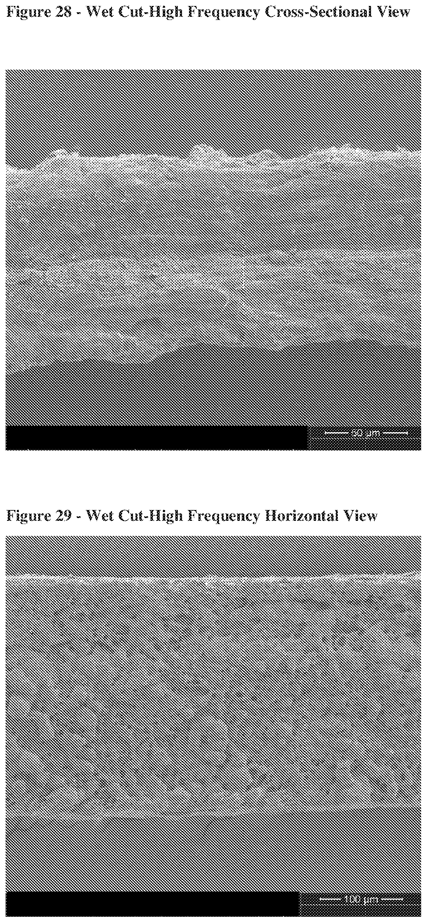

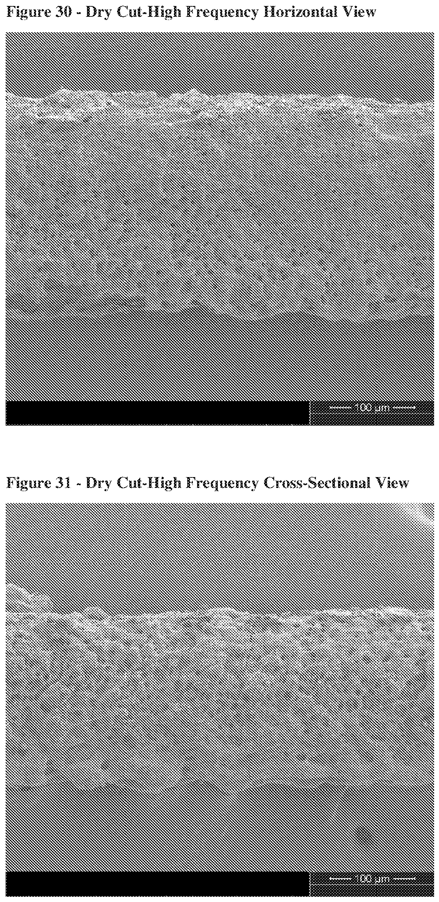

SEM images of the cut filaments were taken from two different perspectives, a horizontal view of the cut surface on both sides and a cross-sectional view of the cut edge. Representative images are provided in Figures 24-31. It was discovered that the laser produced undulating edges with alternating peaks and valleys that were most prominent in the Wet-Low Frequency (W-LF) sample (see Figure 24 and 25). Although strongest along the edge, there was a slight LF distortion within that sample as well. However, it was still possible to observe the smoother, centrally-located ECM surface from the more fibrous cross-section. On other samples, the laser appeared to distort the sample (giving it a melted appearance) such that undulating edge features in the structures were partially smoothed over (see e.g. Figures 26-31) but an undulating edge was still present. The samples cut while dry tended to have more heat-induced laser damage as evidenced by the presence of thicker edges on the cut filaments that tended to curl (e.g. compare Figures for dry cut samples to Figures for wet cut samples.

The use of the terms "a" and "an" and "the" and similar references in the context of describing the invention (especially in the context of the following claims) are to be construed to cover both the singular and the plural, unless otherwise indicated herein or clearly contradicted by context. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

## Claims

1. A filamentous tissue graft, comprising:
an elongate filament body composed of one or more intact segments of a decellularized collagenous tissue membrane isolated from an animal source tissue, wherein the filament body has a length of at least about 20cm and a maximum cross-sectional dimension no greater than about 2mm;
wherein the filament body comprises a laminate of two or more of said intact segments of decellularized collagenous tissue membrane.

2. The tissue graft of claim 1, wherein the one or more intact segments of decellularized collagenous tissue membrane retain collagen, elastin, and bioactive substances native to the animal source tissue, and wherein the bioactive substances include glycosaminoglycans, proteoglycans and growth factors.

3. The tissue graft of any preceding claim, wherein the filament body comprises a laminate of two to twenty of said intact segments of decellularized collagenous tissue membrane.

4. The tissue graft of any preceding claim, also comprising a radiopaque agent carried by the filament body; and optionally wherein the filament body is coated and/or impregnated with a polymeric substance that retards leaching of the radiopaque agent from the filament body when contacted with blood.

5. The tissue graft of claim 4, wherein the radiopaque agent comprises iodine, optionally wherein the radiopaque agent comprises molecular iodine or an iodinated organic compound.

6. The tissue graft of any preceding claim, wherein:
(i) the body has a first outermost face provided by a surface of a first one of said intact segments, a second outermost face opposite the first outermost face and provided by a surface of a second one of said intact segments, a first edge extending between the first outermost face and the second outermost face and provided by a first group of cut edges of the intact segments, and a second edge extending between the first outermost face and the second outermost face and provided by a second group of cut edges of the intact segments; and/or
(ii) the filament body has a length of at least one meter; and/or
(iii) the filament body has an average width in the range of 0.5 mm to 2 mm.

7. The tissue graft of any preceding claim, wherein the filament body has first and second opposed faces and first and second opposed edges extending between the first and second opposed faces, wherein the first and second opposed edges comprise denatured collagenous regions of the intact segments,, optionally wherein the filament body has undenatured collagenous regions of the intact segments extending between and separating the first and second opposed edges comprised of denatured collagenous regions.

8. The tissue graft of any preceding claim, wherein:
(i) the filament body has first and second opposed edges, and wherein the first and second opposed edges are undulating; and/or
(ii) the intact segments retain a nonrandom collagen fibril orientation from the animal source tissue.

9. The tissue graft of any preceding claim, wherein;
(i) the one or more intact segments have been volumetrically expanded relative to a native state of the collagenous tissue membrane in the animal source tissue; and or
(ii) the filament body has a first filament body edge and a second filament body edge, and wherein the first and second filament body edges are defined by alternating peaks and valleys; and/or
(iii) the intact segment(s) of a decellularized collagenous tissue membrane include at least a portion retaining microarchitecture of collagen fibers native to a source tissue for the tissue membrane, optionally wherein the orientation of the collagen fibers of the microarchitecture changes along the length of the filament body.

10. A method for making a filamentous graft material, comprising:
cutting a sheet material comprising a decellularized collagenous tissue membrane isolated from an animal source tissue to form a continuous filament body having a length of at least about 20 cm and a maximum cross-sectional dimension no greater than about 2 mm.

11. The method of claim 10, wherein a) the sheet material comprises a laminate structure including a plurality of decellularized collagenous tissue membrane segments laminated to one another; and / or b) wherein said cutting comprises laser cutting.

12. The method of claim 11, also comprising heating the laminate structure by said cutting, optionally wherein said heating is effective to form an edge region of expanded and fused collagen on the filament body.

13. The method of any one of claim 11 or 12, wherein the sheet material is
(i) wet during said cutting; or
(ii) contains frozen liquid during said cutting, preferably wherein the liquid is an aqueous liquid.

14. The method of any one of claims 11 to 13, also comprising forming, by said cutting, first and second lateral edges and first and second denatured collagenous regions extending inward from the first and second lateral edges, respectively, optionally also comprising leaving, by said cutting, an undenatured collagenous region extending between and separating the first and second denatured collagenous regions, and optionally further wherein the undenatured collagenous region has a thickness that is less than that of the first and second denatured collagenous regions.

15. The method of any one of claim 14, wherein:
a) the sheet material is a generally planar sheet material; or
b) wherein the sheet material is a tubular sheet material, optionally wherein the method comprises rotating the tubular sheet material during said cutting, and optionally further wherein said cutting comprises cutting the tubular sheet material in a helical pattern to form the continuous filament body.

16. The method of any one of claim 14 or 15, wherein the cutting comprises applying a series of discrete but overlapping cutting forces to the sheet material, wherein each of the discrete but overlapping cutting forces removes an amount of the sheet material, optionally wherein the cutting forces comprise laser pulses.

17. The method of any one of claims 14 to 16, wherein the decellularized collagenous tissue membrane retains at least one growth factor from the animal source tissue, optionally wherein said cutting is performed such that the filament body includes an amount of the growth factor.

18. The method of any one of claims 14 to 17, wherein said continuous filament body has a length of at least one meter.

19. The method of any one of claims 14 to 18, wherein the sheet material comprises a radiopaque agent, and wherein the filament body thereby also comprises the radiopaque agent, optionally wherein the radiopaque agent comprises iodine; and optionally further wherein the radiopaque agent comprises molecular iodine or an iodinated organic compound.

20. A filamentous tissue graft of any of claims 1 to 9, which is a multifilament tissue graft including said elongate filament body as a first filament body, and at least a second filament body.

21. The filamentous tissue graft of claim 20, wherein the second filament body is comprised of a different material than the first filament body; optionally wherein the second filament body comprises a metal, optionally wherein the metal is a radiopaque metal, and optionally further wherein the metal is gold.

22. The method of claim 10, wherein
(i) the sheet material is wet during said cutting; or
(ii) the sheet material contains frozen liquid during said cutting, preferably wherein the liquid is an aqueous liquid; or
(iii) the method also comprises forming, by said cutting, first and second lateral edges and first and second denatured collagenous regions extending inward from the first and second lateral edges, respectively, and optionally wherein the method also comprises leaving, by said cutting, an undenatured collagenous region extending between and separating the first and second denatured collagenous regions, and optionally further wherein the undenatured collagenous region has a thickness that is less than that of the first and second denatured collagenous regions.

## Patentansprüche

1. Filamentöses Gewebetransplantat, Folgendes beinhaltend:
einen länglichen Filamentkörper, bestehend aus einem oder mehreren intakten Segment(en) einer dezellularisierten Kollagengewebemembran, welche von einem tierischen Ursprungsgewebe isoliert ist, wobei der Filamentkörper eine Länge von ungefähr 20 cm und eine maximale Querschnittsabmessung von nicht über ungefähr 2 mm aufweist;
wobei der Filamentkörper ein Laminat aus zwei oder mehr der intakten Segmente einer dezellularisierten Kollagengewebemembran beinhaltet.

2. Gewebetransplantat nach Anspruch 1, bei welchem das eine oder die mehreren intakte(n) Segment(e) einer dezellularisierten Kollagengewebemembran Kollagen, Elastin und native bioaktive Substanzen des tierischen Ursprungsgewebes beibehalten, und wobei die bioaktiven Substanzen Glykosaminoglykane, Proteoglykane und Wachstumsfaktoren umfassen.

3. Gewebetransplantat nach einem der vorhergehenden Ansprüche, bei welchem der Filamentkörper ein Laminat aus zwei bis zwanzig der intakten Segmente einer dezellularisierten Kollagengewebemembran beinhaltet.

4. Gewebetransplantat nach einem der vorhergehenden Ansprüche, zudem beinhaltend einen strahlenundurchlässige Wirkstoff, welcher durch den Filamentkörper getragen wird; und optionsweise wobei der Filamentkörper mit einer Polymersubstanz beschichtet und/oder imprägniert ist, welche ein Auslaugen des strahlenundurchlässigen Wirkstoffs aus dem Filamentkörper bei Kontakt mit Blut verzögert.

5. Gewebetransplantat nach Anspruch 4, bei welchem der strahlenundurchlässige Wirkstoff Jod beinhaltet, optionsweise wobei der strahlenundurchlässige Wirkstoff molekulares Jod oder eine jodierte organische Verbindung beinhaltet.

6. Gewebetransplantat nach einem der vorherigen Ansprüche, bei welchem:
(i) der Körper eine erste äußerste Seite besitzt, welche durch eine Oberfläche eines ersten der intakten Segmente bereitgestellt wird, eine zweite äußerste Seite gegenüber der ersten äußersten Seite, welche durch eine Oberfläche eines zweiten der intakten Segmente bereitgestellt wird, eine erste Kante, welche sich zwischen der ersten äußersten Seite und der zweiten äußersten Seite erstreckt und durch eine erste Gruppe von geschnittenen Kanten der intakten Segmente bereitgestellt wird, und eine zweite Kante, welche sich zwischen der ersten äußersten Seite und der zweiten äußersten Seite erstreckt und durch eine zweite Gruppe von geschnittenen Kanten der intakten Segmente bereitgestellt wird; und/oder
(ii) der Filamentkörper eine Länge von mindestens einem Meter aufweist; und/oder
(iii) der Filamentkörper eine mittlere Breite in dem Bereich von 0,5 mm bis 2 mm aufweist.

7. Gewebetransplantat nach einem der vorherigen Ansprüche, bei welchem der Filamentkörper eine erste und eine zweite einander gegenüberliegende Seite und eine erste und eine zweite einander gegenüberliegende Kante besitzt, welche sich zwischen der ersten und der zweiten einander gegenüberliegenden Seite erstrecken, wobei die erste und die zweite einander gegenüberliegende Kante denaturierte Kollagenbereiche der intakten Segmente beinhalten, optionsweise wobei der Filamentkörper undenaturierte Kollagenbereiche der intakten Segmente aufweist, welche sich zwischen der ersten und der zweiten einander gegenüberliegenden Kante erstrecken, welche aus denaturierten Kollagenbereichen bestehen, und diese voneinander trennen.

8. Gewebetransplantat nach einem der vorherigen Ansprüche, bei welchem:
(i) der Filamentkörper eine erste und eine zweite einander gegenüberliegende Kante aufweist, und wobei die erste und die zweite einander gegenüberliegende Kante gewellt sind; und/oder
(ii) die intakten Segmente eine nicht zufällige Ausrichtung der Kollagenfibrillen von dem tierischen Ursprungsgewebe beibehalten.

9. Gewebetransplantat nach einem der vorherigen Ansprüche, bei welchem:
(i) das eine oder die mehreren intakte(n) Segment(e) volumetrisch in Bezug auf einen nativen Zustand der Kollagenmembran in dem tierischen Ursprungsgewebe expandiert wurde(n); und/oder
(ii) der Filamentkörper eine erste Filamentkörperkante und eine zweite Filamentkörperkante aufweist, und wobei die erste und die zweite Filamentkörperkante durch abwechselnde Gipfel und Täler definiert sind; und/oder
(iii) das/die intakte(n) Segment(e) einer dezellularisierten Kollagengewebemembran mindestens einen Abschnitt umfassen, welcher eine native Kollagenfaser-Mikroarchitektur eines Ursprungsgewebes für die Gewebemembran beibehält, optionsweise wobei die Ausrichtung der Kollagenfaser der Mikroarchitektur sich entlang der Länge des Filamentkörpers ändert.

10. Verfahren zur Herstellung eines filamentösen Transplantatmaterials, Folgendes beinhaltend:
Schneiden eines Folienmaterials, beinhaltend eine dezellularisierte Kollagengewebemembran, welche von einem tierischen Ursprungsgewebe isoliert ist, zum Bilden eines kontinuierlichen Filamentkörpers, welcher eine Länge von ungefähr 20 cm und eine maximale Querschnittsabmessung von nicht über ungefähr 2 mm aufweist;

11. Verfahren nach Anspruch 10, bei welchem a) das Folienmaterial eine Laminatstruktur beinhaltet, umfassend eine Vielzahl von aneinander laminierten, dezellularisierten Kollagengewebemembran-Segmenten; und/oder b) wobei das Schneiden Laserschneiden beinhaltet.

12. Verfahren nach Anspruch 11, ebenso beinhaltend Erhitzen der Laminatstruktur durch das Schneiden, optionsweise wobei das Erhitzen effektiv ist, um einen Kantenbereich von expandiertem und geschmolzenem Kollagen an dem Filamentkörper zu bilden.

13. Verfahren nach einem der Ansprüche 11 oder 12, bei welchem das Folienmaterial
(i) feucht während des Schneidens ist; oder
(ii) eine gefrorene Flüssigkeit während des Schneidens enthält, vorzugsweise wobei die Flüssigkeit eine wässrige Flüssigkeit ist.

14. Verfahren nach einem der Ansprüche 11 bis 13, ebenso beinhaltend das Bilden, durch das Schneiden, einer ersten und einer zweiten seitlichen Kante und eines ersten und eines zweiten denaturierten Kollagenbereichs, welche sich jeweils einwärts von der ersten und der zweiten seitlichen Kante erstrecken, optionsweise ebenfalls beinhaltend das Zurücklassen, durch das Schneiden, eines undenaturierten Kollagenbereichs, welcher sich zwischen dem ersten und dem zweiten denaturierten Kollagenbereich erstreckt und diese voneinander trennt, und optionsweise zudem wobei der undenaturierte Kollagenbereich eine Dicke aufweist, welche geringer ist als die des ersten und des zweiten denaturierten Kollagenbereichs.

15. Verfahren nach Anspruch 14, bei welchem:
a) das Folienmaterial ein allgemein planares Folienmaterial ist; oder
b) wobei das Folienmaterial ein röhrenförmiges Folienmaterial ist, optionsweise wobei das Verfahren das Drehen des röhrenförmigen Folienmaterials während des Schneidens beinhaltet, und optionsweise zudem wobei das Schneiden das Schneiden des röhrenförmigen Folienmaterials in einem spiralförmigen Muster zum Bilden des kontinuierlichen Filamentkörpers beinhaltet.

16. Verfahren nach einem der Ansprüche 14 oder 15, bei welchem das Schneiden Aufbringen einer Reihe von diskreten, jedoch überlappenden Schneidkräften auf das Folienmaterial beinhaltet, wobei jede der diskreten jedoch überlappenden Schneidkräfte eine Menge an Folienmaterial entfernt, optionsweise wobei die Schneidkräfte Laserimpulse beinhalten.

17. Verfahren nach einem der Ansprüche 14 bis 16, bei welchem die dezellularisierte Kollagengewebemembran mindestens einen Wachstumsfaktor von dem tierischen Ursprungsgewebe beibehält, optionsweise wobei das Schneiden in einer Weise durchgeführt wird, dass der Filamentkörper eine Menge des Wachstumsfaktors umfasst.

18. Verfahren nach einem der Ansprüche 14 bis 17, bei welchem der kontinuierliche Filamentkörper eine Länge von mindestens einem Meter aufweist.

19. Verfahren nach einem der Ansprüche 14 bis 18, bei welchem das Folienmaterial einen strahlenundurchlässigen Wirkstoff beinhaltet, und wobei der Filamentkörper folglich ebenfalls einen strahlenundurchlässigen Wirkstoff beinhaltet und optionsweise wobei der Filamentkörper Jod beinhaltet; und optionsweise wobei zudem optionsweise der strahlenundurchlässige Wirkstoff molekulares Jod oder eine jodierte organische Verbindung beinhaltet.

20. Filamentöses Gewebetransplantat nach einem der Ansprüche 1 bis 9, welches ein Multifilament-Gewebetransplantat ist, umfassend den länglichen Filamentkörper als einen ersten Filamentkörper und mindestens einen zweiten Filamentkörper.

21. Filamentöses Gewebetransplantat nach Anspruch 20, bei welchen der zweite Filamentkörper aus einem Material besteht, welches sich von dem des ersten Filamentkörpers unterscheidet; optionsweise wobei der zweite Filamentkörper ein Metall beinhaltet, optionsweise wobei das Metall ein strahlenundurchlässiges Metall ist, und optionsweise zudem wobei das Metall Gold ist.

22. Verfahren nach Anspruch 10, bei welchem
(i) das Folienmaterial während des Schneidens feucht ist; oder
(ii) das Folienmaterial eine gefrorene Flüssigkeit während des Schneidens enthält, vorzugsweise wobei die Flüssigkeit eine wässrige Flüssigkeit ist; oder
(iii) das Verfahren ebenso das Bilden, durch das Schneiden, einer ersten und einer zweiten seitlichen Kante und eines ersten und eines zweiten denaturierten Kollagenbereichs beinhaltet, welche sich jeweils einwärts von der ersten und der zweiten seitlichen Kante erstrecken, und optionsweise wobei das Verfahren ebenfalls das Zurücklassen, durch das Schneiden, eines undenaturierten Kollagenbereichs beinhaltet, welcher sich zwischen dem ersten und dem zweiten denaturierten Kollagenbereich erstreckt und diese voneinander trennt, und optionsweise zudem wobei der undenaturierte Kollagenbereich eine Dicke aufweist, welche geringer ist als die des ersten und des zweiten denaturierten Kollagenbereichs.

## Revendications

1. Greffe de tissu filamenteux, comprenant:
un corps filamenteux allongé, composé d'un ou de plusieurs segment(s) intact(s) d'une membrane de tissu collagène décellularisée isolée d'un tissu source animal, dans lequel le corps filamenteux présente une longueur d'au moins environ 20 cm et une dimension en coupe transversale maximale ne dépassant pas environ 2 mm ;
dans lequel le corps filamenteux comprend un stratifié de deux ou plusieurs desdits segments intacts de membrane de tissu collagène décellularisée.

2. Greffe de tissu selon la revendication 1, dans laquelle le ou les segment(s) intact(s) de membrane de tissu collagène décellularisée retiennent le collagène, l'élastine, et des substances bioactives natives du tissu source animal, et dans laquelle les substances bioactives incluent des glycosaminoglycanes, des protéoglycanes et des facteurs de croissance.

3. Greffe de tissu selon l'une quelconque des revendications précédentes, dans laquelle le corps filamenteux comprend un stratifié de deux à vingt desdits segments intacts de membrane de tissu collagène décellularisée.

4. Greffe de tissu selon l'une quelconque des revendications précédentes, comprenant également un agent radio-opaque porté par le corps filamenteux ; et optionnellement dans laquelle le corps filamenteux est revêtu et/ou imprégné d'une substance polymère qui retarde la lixiviation de l'agent radio-opaque du corps filamenteux une fois en contact avec le sang.

5. Greffe de tissu selon la revendication 4, dans laquelle l'agent radio-opaque comprend de l'iode, optionnellement dans laquelle l'agent radio-opaque comprend de l'iode moléculaire ou un composé organique iodé.

6. Greffe de tissu selon l'une quelconque des revendications précédentes, dans laquelle :
(i) le corps présente une première face la plus à l'extérieur fournie par une surface d'un premier desdits segments intacts, une seconde face la plus à l'extérieur opposée à la première face la plus à l'extérieur et fournie par une surface d'un second desdits segments intacts, un premier bord s'étendant entre la première face la plus à l'extérieur et la seconde face la plus à l'extérieur et fourni par un premier groupe de bords de coupe des segments intacts, et un second bord s'étendant entre la première face la plus à l'extérieur et la seconde face la plus à l'extérieur et fourni par un second groupe de bords de coupe des segments intacts, et/ou
(ii) le corps filamenteux présente une longueur d'au moins un mètre ; et/ou
(iii) le corps filamenteux présente une largeur moyenne située dans la plage comprise entre 0,5 mm et 2 mm.

7. Greffe de tissu selon l'une quelconque des revendications précédentes, dans laquelle le corps filamenteux présente une première et une seconde face opposée et un premier et un second bord opposé s'étendant entre les première et seconde face opposées, dans laquelle les premier et second bord opposés comprennent des régions de collagène dénaturées des segments intacts, optionnellement dans laquelle le corps filamenteux présente des régions de collagène non-dénaturées des segments intacts s'étendant entre et séparant les premier et second bords opposés comprenant des régions de collagène dénaturées.

8. Greffe de tissu selon l'une quelconque des revendications précédentes, dans laquelle:
(i) le corps filamenteux présente un premier et un second bord opposé, et dans laquelle les premier et second bords opposés ondulent ; et/ou
(ii) les segments intacts conservent une orientation de fibrille de collagène non-aléatoire à partir du tissu source animal.

9. Greffe de tissu selon l'une quelconque des revendications précédentes, dans laquelle :
(i) le ou les plusieurs segment(s) intact(s) ont été étendu(s) de manière volumétrique par rapport à un état natif de la membrane de tissu de collagène dans le tissu source animal ; et/ou
(ii) le corps filamenteux présente un premier bord de corps filamenteux et un second bord de corps filamenteux, et dans laquelle les premier et second bords de corps filamenteux sont définis par une alternance de pics et de vallées ; et/ou
(iii) le(s) segment(s) intact(s) d'une membrane de tissu de collagène décellularisée incluent au moins une partie conservant une microarchitecture de fibres de collagène native d'un tissu source pour la membrane de tissu, optionnellement dans laquelle l'orientation des fibres de collagène de la microarchitecture change sur la longueur du corps filamenteux.

10. Procédé de réalisation d'un matériau de greffon filamenteux, comprenant :
la découpe d'un matériau en feuille comprenant une membrane de tissu collagène décellularisée isolée d'un tissu source animal afin de former un corps filamenteux continu présentant une longueur d'au moins environ 20 cm et une dimension en coupe transversale maximale ne dépassant pas environ 2 mm.

11. Procédé selon la revendication 10, dans lequel a) le matériau en feuille comprend une structure stratifiée incluant une pluralité de segments de membrane de tissu collagène décellularisée stratifiés les uns sur les autres ; et/ou b) dans lequel ladite découpe comprend la découpe au laser.

12. Procédé selon la revendication 11, comprenant également le chauffage de la structure stratifiée par ladite découpe, optionnellement dans lequel ledit chauffage est efficace afin de former une région de bord de collagène expansé et fondu sur le corps filamenteux.

13. Procédé selon l'une quelconque des revendications 11 ou 12, dans lequel le matériau en feuille est :
(i) humide pendant ladite découpe ; ou
(ii) contient du liquide gelé pendant ladite découpe, de préférence dans lequel le liquide est un liquide aqueux.

14. Procédé selon l'une quelconque des revendications 11 à 13, comprenant également la formation, par ladite découpe, de premier et second bord latéraux et de première et seconde régions collagène dénaturées s'étendant vers l'intérieur depuis les premier et second bords latéraux, respectivement, optionnellement comprenant également le fait de laisser, du fait de ladite découpe, une région collagène non-dénaturée s'étendant entre et séparant les première et la seconde régions collagène dénaturées, et optionnellement en outre dans lequel la région collagène non-dénaturée présente une épaisseur qui est inférieure à celle de la première et de la seconde région collagène dénaturées.

15. Procédé selon la revendication 14, dans lequel :
a) le matériau en feuille est un matériau en feuille généralement plan ; ou
b) dans lequel le matériau en feuille est un matériau en feuille tubulaire, éventuellement dans lequel le procédé comprend la rotation du matériau en feuille tubulaire pendant ladite découpe, et éventuellement en outre dans lequel ladite découpe comprend la découpe du matériau en feuille tubulaire sous un modèle hélicoïdal afin de former le corps filamenteux continu.

16. Procédé selon l'une quelconque des revendications 14 ou 15, dans lequel la découpe comprend l'application d'une série de forces de découpe discrètes mais de chevauchement au matériau en feuille, dans lequel chacune des forces de découpe discrètes mais de chevauchement élimine une quantité du matériau en feuille, optionnellement dans lequel les forces de découpe comprennent des impulsions laser.

17. Procédé selon l'une quelconque des revendications 14 à 16, dans lequel la membrane de tissu collagène décellularisée conserve au moins un facteur de croissance du tissu source animal, optionnellement dans lequel ladite découpe est réalisée de sorte que le corps filamenteux inclue une quantité du facteur de croissance.

18. Procédé selon l'une quelconque des revendications 14 à 17, dans lequel ledit corps filamenteux continu présente une longueur d'au moins un mètre.

19. Procédé selon l'une quelconque des revendications 14 à 18, dans lequel le matériau en feuille comprend un agent radio-opaque, et dans lequel le corps filamenteux comprend également ainsi l'agent radio-opaque, optionnellement dans lequel l'agent radio-opaque comprend de l'iode ; et optionnellement en outre dans lequel l'agent radio-opaque comprend de l'iode moléculaire ou un composé organique iodé.

20. Greffe de tissu filamenteux selon l'une quelconque des revendications 1 à 9, qui est une greffe de tissu multi-filament incluant ledit corps filamenteux allongé en tant qu'un premier corps filamenteux et au moins un second corps filamenteux.

21. Greffe de tissu filamenteux selon la revendication 20, dans lequel le second corps filamenteux se compose d'un matériau différent du premier corps filamenteux ; éventuellement dans lequel le second corps filamenteux comprend un métal, éventuellement dans lequel le métal est un métal radio-opaque, et éventuellement en outre dans lequel le métal est de l'or.

22. Procédé selon la revendication 10, dans lequel :
(i) le matériau en feuille est humide pendant ladite découpe ; ou
(ii) le matériau en feuille contient un liquide gelé pendant ladite découpe, de préférence dans lequel le liquide est un liquide aqueux ; ou
(iii) le procédé comprend également la formation, par ladite découpe, d'un premier et d'un second bord latéral et d'une première et d'une seconde région collagène dénaturée s'étendant vers l'intérieur depuis les premier et second bords latéraux, respectivement, et éventuellement dans lequel le procédé comprend également le fait de laisser, du fait de ladite découpe, une région collagène non-dénaturée s'étendant entre et séparant la première et la seconde région collagène dénaturée, et éventuellement en outre dans lequel la région collagène non-dénaturée présente une épaisseur inférieure à celle de la première et de la seconde région collagène dénaturée.
